# FASCICULE DE BREVET EUROPEEN

(11) **EP 2 903 973 B1**
(45) Date de publication et mention de la délivrance du brevet: **28.06.2017**
(21) Numéro de dépôt: 13786688.5
(22) Date de dépôt: 08.10.2013
(51) Int. Cl.: C07D 249/04

(54) **NOUVEAUX AZOTURES, PROCÉDÉS DE FABRICATION ET LEURS APPLICATIONS**
NEUE AZIDE, VERFAHREN ZUR HERSTELLUNG DAVON UND ANWENDUNGEN DAVON
NEW AZIDES, METHODS FOR PRODUCING SAME AND APPLICATIONS THEREOF

(30) Priorité: 08.10.2012 FR 1259571
(43) Date de publication de la demande: 12.08.2015
(73) Titulaire: Commissariat à l'Énergie Atomique et aux Énergies Alternatives, 75015 Paris (FR)
(72) Inventeur: TARAN, Frédéric, F-91190 Gif Sur Yvette (FR); CHAUMONTET, Manon, F-92130 Issy Les Moulineaux (FR); BEVILACQUA, Valentina, F-91120 Palaiseau (FR)
(74) Mandataire: Cabinet Plasseraud
(86) Numéro de dépôt international: PCT/FR2013/052384
(87) Numéro de publication internationale: WO 2014/057201

(56) Documents cités:
- WO-A2-2006/005046
- GILLES GASSER ET AL: "Synthesis, characterisation and bioimaging of a fluorescent rhenium-containing PNA bioconjugate", DALTON TRANSACTIONS, vol. 41, no. 8, 20 décembre 2011 (2011-12-20), pages 2304-2313, XP055051010, ISSN: 1477-9226, DOI: 10.1039/c2dt12114j
- GASSER G ET AL: "Preparation, <99m>Tc-labeling and biodistribution studies of a PNA oligomer containing a new ligand derivative of 2,2'-dipicolylamine", JOURNAL OF INORGANIC BIOCHEMISTRY, ELSEVIER INC, US, vol. 104, no. 11, 31 juillet 2010 (2010-07-31), pages 1133-1140, XP027275472, ISSN: 0162-0134 [extrait le 2010-07-31]
- ANDRÉ NADLER ET AL: "Histidine Analog Amino Acids Providing Metal-Binding Sites Derived from Bioinorganic Model Systems", EUROPEAN JOURNAL OF ORGANIC CHEMISTRY, vol. 2009, no. 27, 1 septembre 2009 (2009-09-01), pages 4593-4599, XP055051013, ISSN: 1434-193X, DOI: 10.1002/ejoc.200900472
- BANERT K ET AL: "Synthesis of azidochloromethane and azidobromomethane", TETRAHEDRON LETTERS, ELSEVIER, AMSTERDAM, NL, vol. 51, no. 21, 26 mai 2010 (2010-05-26), pages 2880-2882, XP027016190, ISSN: 0040-4039 [extrait le 2010-04-20]
- GUI-CHAO KUANG ET AL: "Chelation-Assisted, Copper(II)-Acetate-Accelerated Azide-Alkyne Cycloaddition", THE JOURNAL OF ORGANIC CHEMISTRY, vol. 75, no. 19, 31 août 2010 (2010-08-31) , pages 6540-6548, XP055026461, ISSN: 0022-3263, DOI: 10.1021/jo101305m
- HEATHER A. MICHAELS ET AL: "Ligand-Assisted, Copper(II) Acetate-Accelerated Azide-Alkyne Cycloaddition", CHEMISTRY - AN ASIAN JOURNAL, vol. 6, no. 10, 29 août 2011 (2011-08-29), pages 2825-2834, XP055051015, ISSN: 1861-4728, DOI: 10.1002/asia.201100426
- GILLES GASSER ET AL: "Towards the Preparation of Novel Re/ 99m Tc Tricarbonyl-Containing Peptide Nucleic Acid Bioconjugates", AUSTRALIAN JOURNAL OF CHEMISTRY, vol. 64, no. 3, 1 janvier 2011 (2011-01-01), page 265, XP055090620, ISSN: 0004-9425, DOI: 10.1071/CH11010
- JULIEN VERGNAUD ET AL: "Design of a new oligotriazole peptide nucleic acid analogue (oT-PNA)", TETRAHEDRON LETTERS, PERGAMON, GB, vol. 52, no. 46, 12 septembre 2011 (2011-09-12), pages 6185-6189, XP028317557, ISSN: 0040-4039, DOI: 10.1016/J.TETLET.2011.09.050 [extrait le 2011-09-17]
- WU P ET AL: "Efficiency and Fidelity in a Click-Chemistry Route to Triazole Dendrimers by the Copper(I)-Catalyzed Ligation of Azides and Alkynes", ANGEWANDTE CHEMIE. INTERNATIONAL EDITION, WILEY VCH VERLAG, WEINHEIM, vol. 43, 1 janvier 2004 (2004-01-01), pages 3928-3932, XP002351095, ISSN: 1433-7851, DOI: 10.1002/ANIE.200454078

## Description

La présente invention concerne des molécules possédant dans leur structure une fonction azoture (N₃) et des groupements chimiques permettant la complexation d'un atome de cuivre, des procédés pour leur fabrication et leurs applications.

La réaction « click » azoture/alcyne fait partie des réactions chimiques les plus citées dans la littérature, on dénombre notamment des milliers d'articles scientifiques et plus de 400 brevets sur ses applications. Cette réaction nécessite du cuivre Cu¹⁺ qui agit comme catalyseur. Cependant pour bon nombre d'applications, les conditions expérimentales (notamment conditions de basses concentrations des réactifs) nécessitent en pratique l'utilisation d'excès de cuivre ce qui s'avère préjudiciable lorsque l'on travaille avec des cellules, des biomolécules ou certaines nanoparticules. Le cuivre est en effet toxique pour les cellules et peut dégrader, dénaturer les protéines ou faire perdre les propriétés de certaines nanoparticules (perte de fluorescence de quantum dots par exemple).

Trois solutions à ce problème sont décrites dans la littérature :
La première consiste à utiliser des alcynes cycliques dont la réactivité est suffisante pour s'affranchir d'une catalyse au cuivre. Ces systèmes sont notamment décrits par l'équipe du Pr. Bertozzi. Cette technique a pour principal inconvénient une cinétique relativement lente ce qui peut se traduire par des rendements de couplage faibles en particulier lorsque deux macromolécules doivent être couplées.
La seconde consiste à utiliser des ligands du Cu¹⁺ qui permettent une accélération de la réaction. La cinétique de la réaction étant augmentée, il est alors possible de réduire la quantité de cuivre. Malgré l'utilisation de ligands du cuivre de plus en plus performants, cette technique implique encore des quantités de cuivre trop élevés pour de nombreuses applications, notamment celle impliquant des cellules ou des biomolécules et nanoparticules fragiles.
La troisième consiste à utiliser des azotures ayant une réactivité particulière en raison de leur capacité à lier le cuivre (voir publication 6). Mais cette technique se limite aux azotures présentant deux atomes d'azotes liant le cuivre, dont celui de la fonction azoture, comme montré sur la formule ci-dessous.

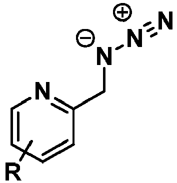

Mais les complexes au cuivre correspondants ne sont donc pas stables ce qui limite leur champ d'application : l'application au ⁶⁴Cu par exemple est impossible et la ligation intracellulaire ou in vivo difficilement envisageable. L'application de ces composés qui est la ligation sur la surface des cellules, nécessite l'addition de ligand du cuivre. Même dans ces conditions, les cinétiques de ligation sont limitées.

Le document Gasser, G. et al. « Synthesis, characterisation and bioimaging of a fluorescent rhenium-cotaining PNA biocojugate », Dalton Transactions, 2011, vol. 41, no. 8, p. 2304-2313, décrit le 2-azido-N,N-bis((quinolin-2-yl)méthyl)éthanamine en tant que ligand pour former un complexe de rhénium qui est ensuite conjugué à un peptide d'acides nucléiques pour former un bio-conjugué.

Le document Gasser, G. et al. « Towards the preparation of Novel Re/99mTc Tricarbonyl-containing peptide nucleic acid Bioconjugates », Australian Journal of Chemistry, 2011, vol. 64, no. 3, p. 265-272, décrit le 2-azido-N,N-bis((pyridin-2-yl)méthyl)éthanamine et le 3-azido-N,N-bis((pyridin-2-yl)méthyl)propanamide. Ce dernier composé est ensuite utilisé dans une réaction click pour former un bio-conjugué pour l'identification d'une cible protéique au sein d'une cellule.

Il serait donc souhaitable de disposer de moyens permettant une réaction « click » azoture/alcyne qui ne nécessite pas de mettre en oeuvre un excès de cuivre.

Il serait également souhaitable de disposer d'une réaction dont la cinétique est suffisamment rapide et dont la biocompatibilité est suffisamment grande pour garder une bonne efficacité même en conditions de haute dilution et en présence de la complexité moléculaire des milieux biologiques .

En résumé, il serait donc souhaitable de disposer de nouveaux réactifs capables de séquestrer le cuivre tout en lui conférant une réactivité accrue pour la réaction « click » azoture/alcyne.

Après de longues recherches, la demanderesse a mis au point un azoture bifonctionnel présentant une structure de ligand du cuivre, qui est capable de jouer le rôle à la fois de réactif et de catalyseur. Il permet de ce fait une réaction « click » azoture/alcyne bi-moléculaire.

C'est pourquoi la présente demande a pour objet un azoture de formule I 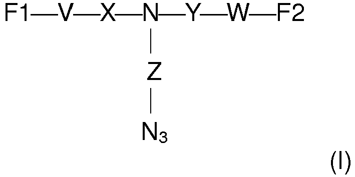 dans laquelle :
V et W représentent indépendamment l'un de l'autre un hétérocycle comprenant un hétéroatome complexant le cuivre, azoté, ou soufré, ledit hétérocycle étant saturé ou insaturé,
X et Y liés à l'azote central représentent indépendamment l'un de l'autre un groupement espaceur de structure -(CH₂)₂-O-(CH₂)-, - (CH₂)-O-(CH₂)₂-, - (CH₂)-O-(CH₂)-, - (CH₂)₂-S- (CH₂)-, - (CH₂)-S-(CH₂)₂-, -(CH₂)-S-(CH₂)-, - (CH₂)₂-NH-(CH₂)-, -(CH₂)-NH-(CH₂)₂-- (CH₂)-NH-(CH₂)-, ou - (CH₂)ₘ-, où m peut prendre les valeurs 1, 2, 3, 4 , de préférence un groupement -(CH₂)ₘ-, où m peut prendre les valeurs 1, 2, 3, 4 ;
Z représente un groupement espaceur, notamment un groupement de structure - (CH₂)₂-O-(CH₂)-, -(CH₂)-O-(CH₂)₂-, - (CH₂)₂-S- (CH₂)-, -(CH₂)-S-(CH₂)₂-, - (CH₂)₂-NH-(CH₂)- -(CH₂)-NH-(CH₂)₂- ou, - (CH₂)ₙ-, où n peut prendre les valeurs 1, 2, 3, 4 ,de préférence un groupement - (CH₂)ₙ-, où n peut prendre les valeurs 1, 2, 3, 4 ;
F1 et F2 représentent indépendamment l'un de l'autre un atome d'hydrogène ou un groupement alkyle tertiaire ayant au plus 7 atomes de carbone, ou un groupement fonctionnel permettant le couplage de l'azoture de formule I à une molécule chimique, à une biomolécule, à une nanoparticule ou à un polymère, , ledit groupement fonctionnel étant un groupement isocyanate, isothiocyanate, carboxyl, amino, maléimide ou thiol, greffé indirectement sur les hétérocycles, étant entendu que au moins l'un de F1 et F2 représente un groupement fonctionnel. F1 et F2 peuvent en plus représenter indépendamment l'un de l'autre un groupement alkyle tertiaire ayant au plus 7 atomes de carbone, de préférence au plus 5 atomes de carbone, notamment un groupement tert-butyl.

Lorsque les hétérocycles sont des hétérocycles azotés, il s'agit avantageusement de la pyridine, l'imidazole, la pyrimidine, la pyrizine, la triazine, l'imidazole, l'azépane, l'oxazole, , le pyrrole, la morpholine, l'isoxazole, l'imidazole, le pyrazole, oxadiazole, le triazole, le tétrazole, le benzimidazole, le benzoxazole, la pyridazine, la quinoléine, l'isoquinoléine, la quinoxaline, la phtalazine, la quinazoline, la benzotriazine, le (1,4,8,11-tetraazacyclotetradecane) (Cyclam) ou le 1,4,7,10-tetraazacyclododecane (Cyclen). Dans cet hétérocycle, l'hétéroatome complexant le cuivre est de préférence espacé par deux à cinq atomes, de préférence de carbone, de l'azote central de l'azoture de formule (I), notamment par deux ou trois atomes, de préférence de carbone.

Lorsque les hétérocycles sont des hétérocycles soufrés, il s'agit avantageusement de l'isothiazole, des thiazoles, du thiophène, du thiadiazole, du benzothiazole ou des thiadiazines

Les hétérocycles sont de préférence choisis parmi les hétérocycles azotés, avantageusement la pyridine, l'imidazole, le pyrrole, le triazole et le benzimidazole.

Comme cela est bien connu de l'homme de l'art, voir par exemple Wikipédia, "Un espaceur moléculaire ou plus simplement un espaceur est dans le domaine de la chimie toute partie variable d'une molécule procurant une connexion entre deux autres parties d'une molécule." Une telle partie peut varier sans changer la fonctionnalité de la molécule en question.

X représente de préférence un groupement méthylène, éthylène ou propylène, particulièrement un groupement méthylène ou éthylène, plus particulièrement un groupement méthylène. Dans un autre mode de réalisation, X représente un groupement éthylène ou propylène.

Y représente de préférence un groupement méthylène, éthylène ou propylène, particulièrement un groupement méthylène ou éthylène, plus particulièrement un groupement méthylène. Dans un autre mode de réalisation, Y représente un groupement éthylène ou propylène.

Z représente de préférence un groupement éthylène, propylène ou butylène, particulièrement un groupement propylène.

L'un au moins de F1 et F2 de préférence représente indépendamment de l'autre un groupement alkyle tertiaire ayant au plus 7 atomes de carbone, de préférence au plus 5 atomes de carbone, notamment un groupement tert-butyl, ou un groupement fonctionnel permettant le couplage de l'azoture de formule (I) à une molécule chimique, une biomolécule, une nanoparticule ou un polymère. F1 et F2 comprennent un groupement isocyanate ou isothiocyanate et de préférence un radical carboxyl, amino, maléimide ou thiol. Ces groupements fonctionnels sont greffés indirectement sur les hétérocycles par exemple par l'intermédiaire d'un groupement alkylène, notamment un groupement alkylène ayant de 1 à 6 atomes de carbone, particulièrement de 2 à 5 atomes de carbone.

Par exemple pour coupler un azoture de formule (I) à une molécule chimique aminée, on choisira par exemple un radical carboxyl et inversement pour coupler un azoture de formule (I) à une molécule chimique comportant un radical carboxyl, on choisira de préférence un radical amino. Ce radical carboxyl trouvera aussi son utilisation par exemple dans le couplage à une biomolécule formée d'acides aminés ou comprenant des acides aminés.

La molécule chimique est de préférence un fluorophore, un ligand, ou un principe actif à usage thérapeutique ou phytosanitaire. Par "ligand" on entend un composé capable de se lier à un récepteur.

La biomolécule est de préférence un anticorps, une protéine, un polysaccharide ou un polynucléotide.

Une nanoparticule est une particule de synthèse dont une dimension est inférieure à 100 nm, et par exemple un nanotube de carbone, une boîte quantique (quantum dot) ou une micelle et, de préférence un quantum dot.

Le polymère est par exemple le polystyrène, le polyacrylamide ou polypropylène et, de préférence le polyacrylamide.

Parmi les azotures de formule I ci-dessus décrits on retient notamment les composés de formule I dans laquelle X et Y représentent indépendamment l'un de l'autre un groupement méthylène, éthylène ou propylène, particulièrement un groupement méthylène ou éthylène, et F1, F2, V, W et Z ont la signification déjà indiquée.

On retient particulièrement les composés de formule I ci-dessus dans laquelle Z représente un groupement - (CH₂)ₙ - où n a la valeur 2 ou 3 et X, Y, F1, F2, V, W et Z ont la signification déjà indiquée.

On retient aussi particulièrement les composés de formule I ci-dessus dans laquelle X et Y représentent indépendamment l'un de l'autre un groupement éthylène ou propylène, Z représente un groupement éthylène ou propylène, et F1, F2, V et W ont la signification déjà indiquée.

On retient plus particulièrement les composés de formule I ci-dessus dans laquelle X et Y représentent indépendamment l'un de l'autre un groupement méthylène ou éthylène, plus particulièrement un groupement méthylène et l'un au moins de F1 et F2 représente indépendamment de l'autre un groupement alkyle tertiaire ayant au plus 7 atomes de carbone, de préférence au plus 5 atomes de carbone, notamment un groupement tert-butyl, ou un groupement fonctionnel, ce groupement fonctionnel étant choisi parmi les groupements fonctionnels spécifiques décrits ci-dessus, c'est-à-dire les groupements isocyanate, isothiocyanate, un radical carboxyl, amino, maléimide ou thiol, et de préférence l'un des quatre derniers groupements fonctionnels cités. Et parmi ceux-ci, on préfère ceux pour lesquelles Z représente un groupement - (CH₂)ₙ-, où n peut prendre les valeurs 1, 2, 3, 4, notamment un radical éthylène, propylène, ou butylène.

On retient encore plus particulièrement, notamment parmi les composés préférés ci-dessus, les composés de formule I ci-dessus dans laquelle V et W représentent indépendamment l'un de l'autre la pyridine, l'imidazole, le pyrrole, le benzimidazole et le triazole, particulièrement ce dernier, X et Y représentent indépendamment l'un de l'autre un groupement méthylène, éthylène ou propylène, particulièrement méthylène ou éthylène, Z représente un groupement éthylène ou propylène, et F1 et F2 ont la signification déjà indiquée.

Parmi les composés particulièrement préférés de l'invention on peut citer de plus
- l'acide 5,5'-(4,4'-(((3-azidopropyl)azanediyl) bis(methylene)) bis(1H-1,2,3-triazole-4,1-diyl)) dipentanoique
- l'acide 5-(4-(((3-azidopropyl)((1-(tert-butyl)-1H-1,2,3-triazol-4-yl)méthyl)amino)méthyl)-1H-1,2,3-triazol-1-yl) pentanoique et
- le 4,4'-(4,4'-(((3-azidopropyl)azanediyl)bis(methylene))bis(1H-1,2,3-triazole-4,1-diyl))bis(N-(3-aminopropyl)butanamide).

A noter que dans la présente demande, classiquement l'article indéfini "un" doit être considéré comme un pluriel générique (signification de "au moins un" ou encore "un ou plusieurs"), sauf lorsque le contexte montre le contraire (1 ou "un seul"). Ainsi, par exemple, lorsque l'on dit ci-dessus que l'on utilise un azoture de formule I, il s'agit de l'utilisation d'un ou plusieurs azotures de formule I.

La présente demande a aussi pour objet un procédé de préparation d'un azoture de formule I ci-dessus, dans laquelle X et Y représentent indépendamment l'un de l'autre un groupement -(CH₂)ₘ -, où m a la valeur 1, 2, 3 ou 4, caractérisé en ce que l'on fait réagir un amino alcool de formule II

OH-Z-NH₂ (II)

dans laquelle Z à la signification déjà indiquée avec un aldéhyde de formule III

GP₁-F₁-V-X'-CHO (III)

dans laquelle F₁ et V ont la signification déjà indiquée, GP₁ représente un groupement protecteur d'une fonction F1 et X' représente un groupement - (CH₂)ₘ₋₁ - où m a la valeur déjà indiquée,
pour obtenir un composé de formule IV

GP₁-F₁-V-X'-CH₂-NH-Z-OH (IV)

dans laquelle GP₁, F₁, V, X' et Z ont la signification déjà indiquée,
que l'on soumet, avec un aldéhyde de formule V

GP₂-F₂-W-Y'-CHO (V)

dans laquelle F₂ et W ont la signification déjà indiquée, GP₂ représente un groupement protecteur d'une fonction F2 et Y' représente représente un groupement - (CH₂)ₘ₋₁ - où m a la valeur déjà indiquée,
à une amination réductrice pour obtenir un composé de formule VI 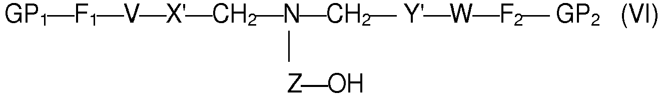 dans laquelle GP₁, F₁, V, X', Y', W, F₂, GP₂ et Z ont la signification déjà indiquée, que l'on fait réagir avec un azoture alcalin de formule VII

EN₃ (VII)

dans laquelle E représente un métal alcalin, de préférence le sodium,
pour obtenir un composé de formule VIII 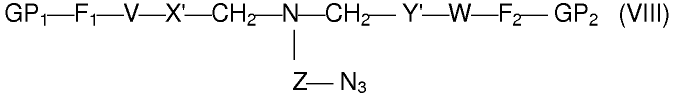 dans laquelle GP₁, F₁, V, X', Y', W, F₂, GP₂ et Z ont la signification déjà indiquée, dont on élimine les groupement protecteurs pour obtenir le composé de formule IA 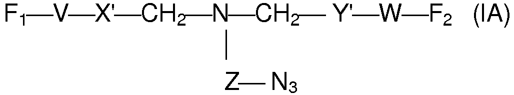 attendu que l'on isole si désiré.

Dans des conditions préférentielles de mise en oeuvre du procédé ci-dessus décrit,
- la réaction d'amination réductrice est effectuée en présence d'un hydrure;
- pour faciliter la réaction du composé de formule VI avec un azoture alcalin de formule VII, on active la fonction alcool du composé de formule VI par un groupement tosyle, mésyle ou triflate, de préférence un groupement mésyle;
- l'élimination des groupements protecteurs GP₁ et GP₂ est de préférence effectuée à l'aide d'une solution de soude aqueuse dans le cas d'une fonction F₁ ou F₂ acide carboxylique protégée sous forme d'un ester, et à l'aide d'acide trifluoroacétique dans le cas d'une fonction amine protégée sous forme de carbamate.

En résumé, la voie de synthèse ci-dessus consiste à faire réagir un amino-alcool, dont les fonctions amine et alcool sont séparées par un espaceur Z, avec un composé présentant une fonction aldéhyde, un espaceur X' (correspondant à l'homologue inférieur de l'espaceur X - perte d'un chaînon de l'espaceur X), un hétérocycle V, une fonction F₁ protégée par un groupement protecteur GP₁ selon une réaction d'amination réductrice. L'amine secondaire ainsi obtenue peut alors subir une deuxième réaction d'amination réductrice avec un nouveau composé présentant une fonction aldéhyde, un espaceur Y' (correspondant à l'homologue inférieur de l'espaceur Y), un hétérocycle W, une fonction F₂ protégée par un groupement protecteur GP₂. Dans le cas de préparation d'une molécule symétrique (F₁ = F₂, V = W et X = Y), les deux réactions d'amination réductrice peuvent se réaliser en une seule étape, par exemple en utilisant deux équivalents du réactif aldéhyde.

L'étape suivante consiste à activer la fonction alcool par un groupement mésyl de manière à faciliter l'étape de substitution par l'azoture de sodium (NaN₃) pour former l'azoture correspondant.

La présente demande a également pour objet un procédé de préparation d'un azoture de formule I ci-dessus, dans laquelle V et W représentent un triazole, caractérisé en ce que l'on fait réagir un alcyne halogéné de formule Xa 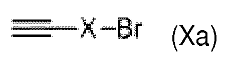 dans laquelle X à la signification déjà indiquée, et Br représente un atome d'halogène, de préférence un atome de brome,
avec un amino alcool de formule XI

OH-Z-NH₂ (XI)

dans laquelle Z à la signification déjà indiquée,
pour obtenir un dialcyne de formule XII 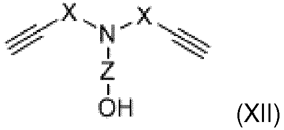 dans laquelle X et Z ont la signification déjà indiquée,
que l'on fait réagir avec un azoture de formule XIII

GP₁-F₁-N₃ (XIII)

dans laquelle GP₁ et F₁ ont la signification déjà indiquée, en présence d'un catalyseur de préférence à base de cuivre,
pour obtenir par cycloaddition un alcyne de formule XIV, 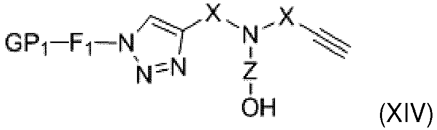 dans laquelle GP₁, F₁, X et Z ont la signification déjà indiquée,
que l'on fait réagir avec un azoture de formule XV

GP₂-F₂-N₃ (XV)

dans laquelle GP₂ et F₂ ont la signification déjà indiquée, en présence d'un catalyseur de préférence à base de cuivre, pour obtenir par cycloaddition un composé de formule XVI, 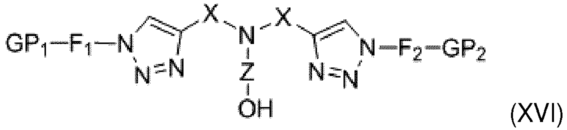 dans laquelle GP₁, GP₂, F₁, F₂, X et Z ont la signification déjà indiquée,
que l'on fait réagir avec un azoture alcalin de formule VII

EN₃ (VII)

dans laquelle E a la signification déjà indiquée,
pour obtenir un composé de formule XVII 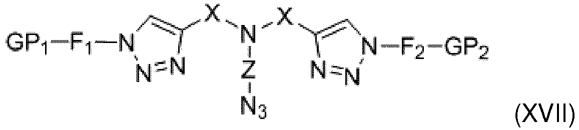 dans laquelle GP₁, GP₂, F₁, F₂, X et Z ont la signification déjà indiquée, dont on élimine les groupement protecteur pour obtenir le composé de formule IB 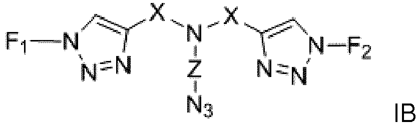 attendu dans laquelle F₁, F₂, X et Z ont la signification déjà indiquée et que l'on isole si désiré.

Dans des conditions préférentielles de mise en oeuvre du procédé ci-dessus décrit
- la réaction d'un alcyne halogéné de formule Xa avec un amino alcool de formule XI est réalisée en présence d'une base et de préférence du carbonate de sodium ou de potassium, sous chauffage modéré et de préférence à 50°C;
- la réaction du dialcyne de formule XII ou du monoalcyne de formule XIV avec un azoture de formule XV est réalisée en présence d'un catalyseur à base de cuivre et de préférence en présence d'un agent réducteur tel que l'acide ascorbique;
- la réaction du composé de formule XVI avec un azoture alcalin de formule VII est réalisée en présence d'un activateur de la fonction alcool tel qu'un halogénure de méthanesulfonyle comme le chlorure de méthanesulfonyle, dans un solvant tel que la triéthylamine.

Les azotures de formule I objet de la présente invention possèdent de très intéressantes propriétés. Ils ont la capacité de complexer le cuivre et de réagir par leur fonction azoture sur des fonctions alcynes terminales selon la réaction de cycloaddition 1,3-dipolaire des azotures sur les alcynes catalysée par le cuivre, réaction appartenant à ce qui est appelé la chimie « Click ».

Les azotures de formule I objet de la présente invention permettent ainsi le couplage covalent (ou ligation) entre deux entités identiques ou différentes (molécules chimiques, nanoparticules, biomolécules, polymères...) ceci avec une grande efficacité même dans des milieux complexes tels que les milieux biologiques comme les milieux de culture et les lysats cellulaires ou le plasma. La ligation est également très chimiosélective, ce qui explique qu'elle peut s'opérer efficacement même en milieux complexes (milieux cellulaires, sang...), voire *in vivo.*

Les azotures de formule I objet de la présente invention sont bifonctionnels car ils jouent le rôle à la fois de réactif et de catalyseur. De ce fait leur réaction avec les entités ci-dessus devient bi-moléculaire (l'entité et l'azoture) ce qui est cinétiquement favorable par rapport aux techniques qui nécessitent la rencontre de trois partenaires.

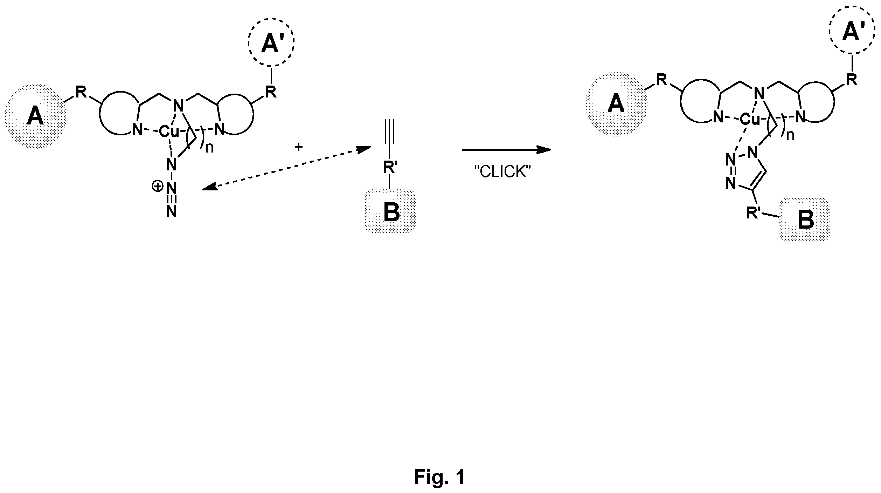

La réactivité des azotures de formule I objet de la présente invention pour les alcynes est telle que la réaction de ligation est extrêmement rapide, par conséquent elle est encore efficace même dans des conditions de haute dilution, ce qui est classiquement le cas pour les bio-conjugaisons par exemple. Par le biais des groupements fonctionnels F1 et F2, les azotures de formule I objet de la présente invention peuvent être facilement marqués par un fluorophore A' puis liés de façon covalente à un anticorps A par exemple. Cet anticorps pourra alors réagir en présence de cuivre avec une nanoparticule B préalablement fonctionnalisée par un alcyne terminal pour conduire au conjugué correspondant comme illustré par un exemple à la figure 1.

Ces propriétés sont illustrées ci-après dans la partie expérimentale. Elles justifient l'utilisation des azotures de formule I ci-dessus décrits, dans la préparation de conjugués molécules-nanoparticules, de conjugués molécules-biomolécules, de conjugués nanoparticules-biomolécules, de conjugués biomolécules-polymères etc... ;

Elles justifient aussi l'utilisation des azotures de formule I ci-dessus décrits, dans les techniques de « fishing » permettant d'isoler et d'identifier une cible protéique au sein de mélanges biologiques complexes ou même dans une cellule notamment humaine. Les techniques de « fishing » sont classiquement réalisées par la technologie streptavidine/biotine (biotine greffée sur la molécule B et streptavidine immobilisée sur le support) dont les limitations dues aux interactions non spécifique de la biotine sont connues. L'utilisation des azotures de la présente invention ne connaît pas une telle limitation.

La technique de « fishing » de la présente invention peut être mise en oeuvre sur des cellules entières, en contraste avec les techniques où il est nécessaire d'appliquer la technique sur lysat cellulaire.

Elles justifient aussi l'utilisation des azotures de formule I ci-dessus décrits, dans l'imagerie par des complexes au ⁶⁴Cu. En effet, Le cuivre reste lié une fois la réaction de ligation effectuée.

La stabilité du complexe au cuivre obtenu avec les azotures de formule I de l'invention permet de conserver une grande efficacité de la réaction de ligation puisque celle-ci est réalisée avec un rendement proche de 100%, contre moins de 20% avec les solutions de l'art antérieur.

C'est pourquoi la présente demande a aussi pour objet l'utilisation des azotures de formule I ci-dessus décrits, notamment dans
- la préparation de bioconjugués ou de nanoconjugués,
- l'isolation ou l'identification d'une cible notamment protéique, particulièrement au sein d'un mélange biologique complexe comme des lysats cellulaires, des cultures cellulaires ou du plasma,
- l'imagerie notamment par des complexes au ⁶⁴Cu.

Pour son utilisation dans la préparation de bioconjugués ou de nanoconjugués, le procédé peut notamment comporter les étapes suivantes :
1) On greffe un azoture de formule I sur une biomolécule ou sur une nanoparticule
2) On greffe un alcyne sur une molécule, une biomolécule ou une nanoparticule
3) On met en contact les deux partenaires azoture et alcyne greffés en présence d'ions cuivreux en conséquence de quoi les dits partenaires se retrouvent liés de façon covalente l'un à l'autre.

Pour son utilisation dans l'isolation ou l'identification d'une cible notamment protéique particulièrement au sein d'un mélange biologique complexe comme du lysat cellulaire, le procédé peut notamment comporter les étapes suivantes :
1) on greffe un azoture de formule I sur un support, par exemple comprenant des billes, sur les parois un récipient ou sur une plaque,
2) on met en contact avec ce support greffé un milieu contenant une molécule B se liant à la cible notamment protéique et comportant une fonction alcyne terminale en présence d'ions cuivreux en conséquence de quoi ledit composé se trouve fixé au support,
3) La cible par exemple protéique ainsi fixée sur le support est digérée par des enzymes de restriction telle que la trypsine et l'analyse des peptides ainsi générés permet l'identification de celle-ci.

Pour son utilisation dans l'imagerie notamment par des complexes au ⁶⁴Cu, le procédé peut notamment comporter les étapes suivantes :
1) on greffe un alcyne sur une biomolécule ou une nanoparticule
2) on prépare un complexe cuivreux ⁶⁴Cu/azoture de formule I
3) on met en contact les deux partenaires en conséquence de quoi les dits partenaires se retrouvent liés de façon covalente l'un à l'autre.

Comme on le verra ci-après dans la partie expérimentale, la structure particulière des azotures de la présente invention permet une réaction "click" extrêmement rapide.

La présente demande décrit également des produits industriels utiles notamment pour la préparation des azotures de formule I ci-dessus, les azotures de formule XX 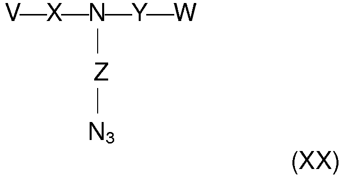 dans laquelle X, Y, V, W et Z ont la signification déjà indiquée pour les azotures de formule I.

Les conditions préférentielles de mise en oeuvre des azotures de formule I ci-dessus décrites s'appliquent également aux autres objets de l'invention visés ci-dessus, notamment à leurs procédés de fabrication, à leurs utilisations et aux azotures de formule XX.

Les exemples et expérimentations qui suivent illustrent la présente demande et l'invention sera mieux comprise si l'on se réfère aux dessins annexés sur lesquels
- la figure 1 représente un premier type d'application des azotures de formule I de la présente invention, à la préparation de conjugués. A et B peuvent être des molécules, des biomolécules, des nanoparticules ou des polymères. A' peut être une molécule telle que par exemple un fluorophore.

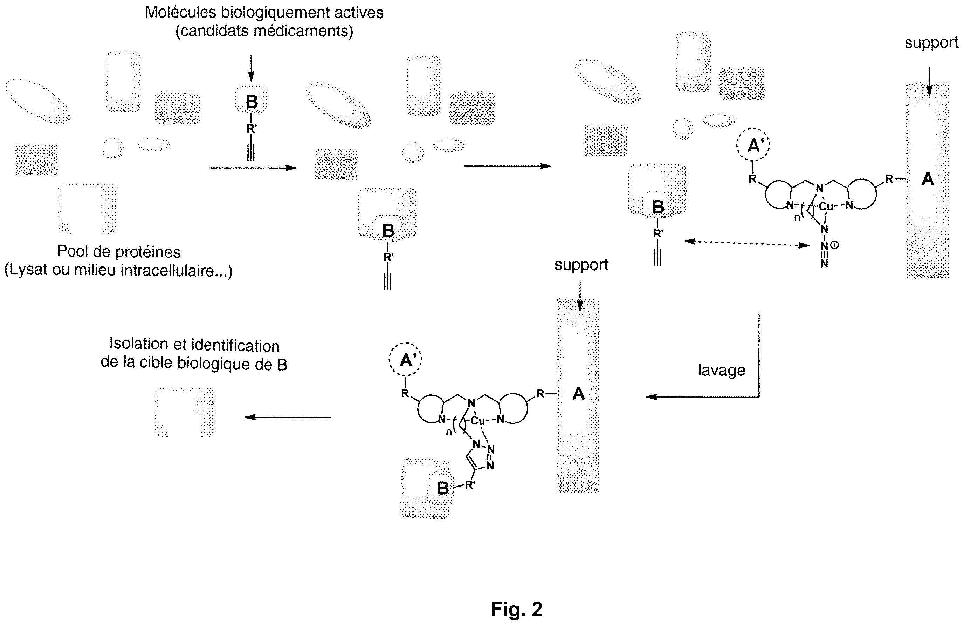

- la figure 2 illustre le principe d'un exemple d'amélioration des techniques de fishing

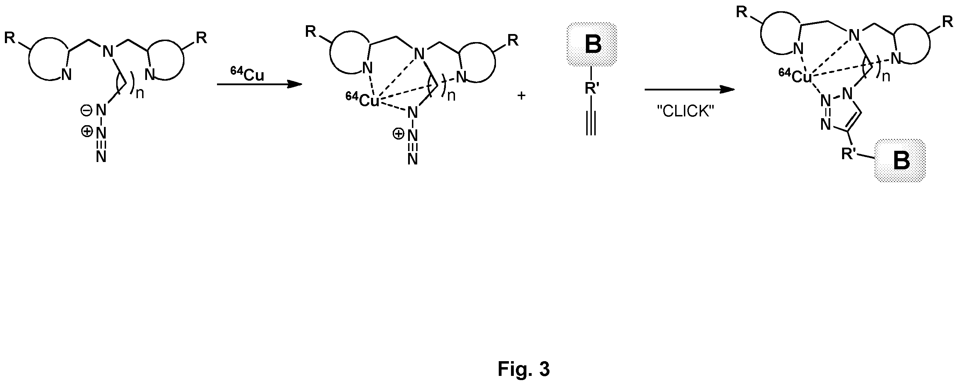

- la figure 3 illustre le principe d'un exemple de développement de complexes radioactifs « clickables ».
- la figure 4 est un graphe représentant le rendement de ligation exprimé en pourcentage en fonction du temps exprimé en minutes obtenu avec des azotures de l'invention et obtenu avec des azotures de l'art antérieur, dans un milieu simple (solution aqueuse tamponnée).
- la figure 5 est un graphe représentant le rendement de ligation exprimé en pourcentage en fonction du temps exprimé en minutes obtenu avec des azotures de l'invention et obtenu avec des azotures de l'art antérieur, dans un milieu complexe.
- la figure 6 est un graphe représentant les cinétiques comparées de réaction click entre différents azotures chélatants dans un milieu tampon phosphate.
- la figure 7 est un graphe représentant les cinétiques comparées de réaction click entre différents azotures chélatants dans un lysat cellulaire.
- la figure 8 représente des images de microscopie confocale de cellules HuH-7.

### Préparation 1 : 3-azido-N,N-bis((1-methyl-1H-benzo[d]imidazol-2-yl)methyl)propan-1-amine (Azoture A1)

### Stade 1 : 3-(bis((1-methyl-1H-benzo[d]imidazol-2-yl)methyl)amino)propan-1-ol

A une solution de méthyl-2-formyl benzimidazole (730 mg, 4,6 mmol) dans le THF anhydride (25mL) sont ajoutés le 3-aminopropanol (174 µL, 2,3 mmol), et de l'acide acétique (260 µL, 4,6 mmol). Le mélange réactionnel est ensuite agité pendant 48h à température ambiante sous atmosphère de N₂. Après concentration du brut réactionnel sous vide, le résidu est repris par du CH₂Cl₂ puis lavé par une solution saturée en bicarbonate de sodium. La phase aqueuse est extraite deux fois au dichlorométhane puis les phase organiques sont regroupées, séchées sur MgSO₄ et concentrées. Le brut réactionnel est enfin purifié par chromatographie flash sur colonne de silice (9/1 AcOEt/MeOH) pour donner le produit attendu sous forme d'une huile incolore (245 mg, 0,68 mmol, 30% de rendement).
RMN ¹H (400MHz *,Chloroforme-d*) δ = 7,70 (m, 2 H), 7,24 - 7,19 (m, 6 H), 3,96 (s, 4 H), 3,82 (t, *J* = 5,8 Hz, 2 H), 3,67 (s, 6 H), 2,92 (t, *J* = 5,8 Hz, 2 H), 1,84 (quin, *J*= 5,8 Hz, 2 H) ppm ;
RMN ¹³C (101MHz, *Chloroforme-d*) δ = 151,5 (2C), 141,7 (2C), 135,9 (2C), 122,9 (2C), 122,3 (2C), 119,4 (2C), 109,3 (2C), 60,8, 53,5, 51,4 (2C), 30,1 (2C), 29,5 ppm ;
IR (pastilles NaCl): 3287, 2938, 1478,1402, 1333, 174 cm⁻¹ ;

### Stade 2 : 3-azido-N,N-bis((1-methyl-1H-benzo[d]imidazol-2-yl)methyl)propan-1-amine

A une solution d'alcool obtenu précédemment (230mg, 0,63 mmol) dans le DMF anhydre sont ajoutés de la triéthylamine (106 µL, 0,76 mmol) et du chlorure de mésyl (59 µL, 0,76 mmol) puis le milieu réactionnel est agité à température ambiante sous atmosphère de N₂. Après disparition de l'alcool de départ (contrôle CCM) l'azoture de sodium (205mg, 3,2 mmol) est ajouté au mélange réactionnel. Après 12h d'agitation le brut réactionnel est concentré sous vide, et directement purifié par chromatographie flash sur colonne de silice (9/1 AcOEt/MeOH) pour donner le produit attendu sous forme d'un solide amorphe blanc (172 mg, 0,44 mmol, 70% de rendement).
RMN ¹H (400MHz *,Chloroforme-d*) δ = 7,75 - 7,71 (m, 2 H), 7,29 - 7,24 (m, 6 H), 4,01 (s, 4 H), 3,63 (s, 6 H), 3,23 (t, *J* = 6,9 Hz, 2 H), 2,84 - 2,79 (m, 2 H), 1,87 - 1,78 (m, 2 H) ppm ;
RMN ¹³C (101MHz *Chloroforme-d*) δ = 151,2 (2C), 142,2 (2C), 136,1 (2C), 123,0 (2C), 122,3 (2C), 119,8 (2C), 109,3 (2C), 52,2, 51,3 (2C), 49,4, 29,9 (2C), 26,2 ppm ;
IR (pastilles NaCl): 3382, 3054, 2946, 2096, 1479, 1333, 1128, 747 cm⁻¹ ; SMHR : pour C₂₁H₂₅N₈⁺ [M+H]⁺ : 389,2206, calc. 389,2202.

### Préparation 2 : 3-azido-N,N-bis((1-benzyl-1H-1,2,3-triazol-4-yl)methyl)propan-1-amine

### Stade 1 : 3-(di(prop-2-yn-1-yl)amino)propan-1-ol

A une solution d'aminopropanol (500 µL, 6,54 mmol) et de carbonate de sodium (2,08 g, 19,64 mmol) dans l'éthanol (20 mL) est ajouté à température ambiante le propargylbromide (1,46 mL, 13,09 mmol) puis le mélange réactionnel est agité la nuit à 50°C. Après retour à température ambiante et évaporation sous pression réduite du mélange réactionnel de l'eau est ajoutée. La phase aqueuse est acidifiée (pH=2-3) par ajout d'une solution de HCl (2N) puis extraite 3 fois par de l'acétate d'éthyle puis les phases organiques sont regroupées, séchées sur MgSO₄ et concentrées. Le produit attendu est obtenu sous forme d'une huile jaunâtre (600 mg, 3,97 mmol, 60% rendement) et engagé directement dans l'étape suivante.

RMN ¹H (400MHz, *Chloroforme-d*) δ = 3,77 (t, 2H, *J*= 5,4 Hz), 3,47 (d, 4H, *J* = 2,4 Hz), 2,78 (t, 2H, *J* = 6,1 Hz), 2,24 (t, 2H, *J* = 2,4 Hz), 1,71 (quint, 2H, *J* = 5,8 Hz) ppm ;

RMN ¹³C (101MHz *,Chloroforme-d*) δ = 78,2 (2C), 79,4 (2C), 63,4 (1C), 52,1 (1C), 42,2 (2C), 28,4 (1C) ppm ;

### Stade 2 : 3-(bis((1-benzyl-1H-1,2,3-triazol-4-yl)methyl)amino)propan-1-ol

Dans un ballon muni d'un agitateur magnétique, dissoudre l'alcyne (9,8 mmol mmol, 1eq) et le benzyl azoture (2 eq) dans un mélange équimolaire H₂O/^{t}BuOH. Le milieu réactionnel est soumis à une agitation constante. L'ascorbate de sodium (9,8 mmol, 1eq) et le sulfate de cuivre pentahydraté (0,98 mmol, 10% mol) sont ajoutés successivement. La réaction est soumise à agitation à température ambiante pendant la nuit. Le brut réactionnel est concentré puis purifié sur colonne ouverte de silice.
Purification : EtOAc / MeOH Rf = 0,35
Rendement : 60 %

RMN ¹H (CDCl3, 400 MHz) : δ (ppm) 7,57 (s, 2H), 7,29 (m, 6H), 7,20 (m, 4H), 5,45 (s, 4H), 3,70 (s, 4H), 3,62 (t, 2H, J = 4,0 Hz), 2,67 (t, 2H, J = 8,0 Hz), 1,71 (t, 2H, J = 8,0 Hz).

RMN ¹³C (CDCl3, 100 MHz) : δ (ppm) 144 (2C), 135 (2C), 129 (2C), 128 (2C), 127 (2C), 123 (2C), 62,8, 53,8 (2C), 52,3 (2C), 47,3, 27,9.

FTIR (neat) v (cm-1) = 3428, 1643, 1456, 1345, 1173, 1051.

SM (ES+) : m/z = 418,8

### Stade 3 : 3-azido-N,N-bis((1-benzyl-1H-1,2,3-triazol-4-yl)methyl)propan-1-amine

A une solution d'alcool obtenu précédemment (82 mg, 0,20 mmol) dans le DMF anhydre sont ajoutés de la triéthylamine (27 µL, 0,20 mmol) et du chlorure de mésyl (15 µL, 0,20 mmol) puis le milieu réactionnel est agité à température ambiante sous atmosphère de N₂. Après disparition de l'alcool de départ (contrôle CCM) l'azoture de sodium (64 mg, 0,98 mmol) est ajouté au mélange réactionnel. Après 12h d'agitation le brut réactionnel est concentré sous vide, et directement purifié par chromatographie flash sur colonne de silice (95/5 AcOEt/MeOH) pour donner le produit attendu sous forme d'une huile transparente (65 mg, 0,15 mmol).
Purification : EtOAc / MeOH Rf = 0,38
Rendement = 75 %

RMN ¹H (CDCl3, 400 MHz) : δ (ppm) 7,51 (s, 2H), 7,34 (m, 6H), 7,25 (m, 4H), 5,49 (s, 4H) δ 3,70 (s, 4H), 3,27 (t, 2H, J = 4 Hz), 2,52 (t, 2H, J = 8 Hz), 1,79 (quint, 2H, J = 8 Hz).

RMN ¹³C (CDCl3, 100 MHz) : δ (ppm) 144 (2C), 134 (2C), 130 (2C), 129 (2C), 128 (2C), 123 (2C), 53,9, 49,1, 47,5, 26,3.

FTIR (neat) v (cm-1 ) = 3428, 1643, 1456, 1345, 1173, 1051

SM (ES+) : m/z = 443,7

### Préparation 3 : 5,5'-(4,4'-(((3-azidopropyl)azanediyl) bis(methylene))bis(1H-1,2,3-triazole-4,1-diyl)) dipentanoate de diéthyle

### Stade 1 : 5,5'-(4,4'-(((3-hydroxypropyl)azanediyl)bis(methylene))bis(1H-1,2,3-triazole-4,1-diyl)) dipentanoate de diéthyle

A une solution de 3-(di(prop-2-yn-1-yl)amino)propan-1-ol 2 (2,88 g, 0,0193 mol) dans 12 mL d'eau / ^{t}BuOH sont ajoutés l' ethyl 5-azidopentanoate (3,264 g, 0,0193 mol), le CuSO₄.5H₂O (482 mg, 1,93 mmol) et l'ascorbate de Sodium (3,82 g, 0,0193 mol). La réaction est soumise à agitation à température ambiante pendant 12h. Le brut réactionnel est évaporé à sec et purifié sur colonne de silice (EtOAc/MeOH) pour obtenir le produit attendu (2,46 g, rendement de 52 %).

RMN ¹H (CDCl3, 400 MHz) : δ (ppm) 7,96 (s, 2H), 4,43 (t, 4H, J = 8,0 Hz), 4,10 (q, 4H, J = 8,0 Hz), 3,76 (s, 4H), 3,59 (t, 2H, J = 8,0 Hz), 3,35 (s, 1 H), 2,58 (t, 2H, J = 8,0 Hz), 2,36 (t, 4H, J = 8,0 Hz), 1,95 (quint, 4H, J = 8,0 Hz), 1,78 (quint, 2H, J = 8,0 Hz), 1,59 (quint, 4H, J = 8,0 Hz), 1,22 (t, 6H, J = 8,0 Hz).

RMN ¹³C (CDCl3, 100 MHz) : δ (ppm) 172 (2C), 143 (2C), 123 (2C), 62,5 (2C), 60,1, 52,1 (2C), 49,8 (2C), 47,1, 33,1 (2C), 29,2, 27,9 (2C), 21,5 (2C), 13,9 (2C).

FTIR (neat) v (cm-1) = 3399, 3139, 2942, 1730, 1459, 1375, 1330, 1256, 1187, 1129, 1052, 790.

SM (ES+) : m/z = 494,8

### Stade 2 : 5,5'-(4,4'-(((3-azidopropyl)azanediyl)bis(methylene))bis(1H-1,2,3-triazole-4,1-diyl)) dipentanoate de diéthyle

A une solution de 5,5'-(4,4'-(((3-hydroxypropyl)azanediyl)bis(methylene))bis(1H-1,2,3-triazole-4,1-diyl))dipentanoate de diéthyle (246 mg, 0,5 mmol) dans du DMF anhydre (10 mL) sont ajoutés la TEA (0,548 mmol), le MsCl (571 mg, 0,274 mmol). Après 3h d'agitation sous atmosphère inerte, on ajoute le NaN₃ (486 mg, 0,41 mmol) et le mélange réactionnel est agité pendant 12h à température ambiante et sous N₂. Le brut réactionnel est concentré sous vide et le produit brut est purifié sur colonne de silice (EtOAc/MeOH) pour donner le produit pur attendu (193 mg, 0,37 mmole, 74% de rendement).

RMN ¹H (400MHz ,Méthanol-*d4*) δ = 7,97 (s, 2H), 4,43 (t, 4H, J = 8 Hz), 4,11 (q, 4H, J = 8 Hz), 3,75 (s, 4H), 2,36 (t, 4H, J = 8 Hz), 1,95 (quint, 4H, J = 8Hz), 1,78 (quint, 2H, J = 8Hz), 1,59 (quint, 4H, J = 8Hz), 1,22 (t, 6H, J = 8Hz) ppm ; RMN ¹³C (101 MHz *,Méthanol-d4*) δ = 175 (2C), 146 (2C), 125 (2C), 61,6 (2C), 51,1, 49,8 (2C), 34,4 (2C), 30,7 (2C), 27,7, 23,0 (2C), 14,7 (2C) ppm ; IR (pastilles NaCl): 3135, 2941, 2097, 1731, 1458, 1374, 1255, 1185, 1047, 822 cm⁻¹ ;

MS (ES+) : m/z = 519,8

### Exemple 1 : acide 5,5'-(4,4'-(((3-azidopropyl)azanediyl) bis(methylene))bis(1H-1,2,3-triazole-4,1-diyl))dipentanoique

A une solution de 5,5'-(4,4'-(((3-azidopropyl)azanediyl) bis(methylene))bis(1H-1,2,3-triazole-4,1-diyl)) dipentanoate de diéthyle (946 mg, 1,825 mmol) de la préparation 3 dans une solution de NaOH (2M, 292 mg), EtOH (3,65 mL) est ajouté pour rendre le milieu réactionnel homogène. La solution est agitée à température ambiante pendant 24h et l'éthanol est évaporé à sec. La phase aqueuse est acidifiée jusqu'à pH = 7 avec une solution de HCl (1 M) et évaporée. Le brut réactionnel est dissous dans du MeOH glacé et les sels sont filtrés 2 fois et on obtient le produit attendu (843 mg, rendement de 99 %).

RMN ¹H (400MHz ,Méthanol-*d4*) δ = 8,00 (s, 2H), 4,44 (t, 4H, J = 8 Hz), 3,76 (s, 4H), 2,72 (t, 2H, J = 8 Hz), 2,23 (t, 4H, J = 8 Hz), 1,96 (quint, 4H, J = 8 Hz), 1,82 (quint, 2H, J = 8 Hz), 1,63 (quint, 4H, J = 8 Hz) ppm ;
IR (pastilles NaCl): 4453, 4197, 3944, 3352, 3055, 2985, 2832, 2522, 2306, 2099, 1571, 1420, 1266, 1222, 1126, 1024, 981, 896, 737, 704 cm⁻¹

Le composé de l'exemple 1 présente deux groupements COOH permettant une fonctionnalisation de l'azoture chélatant. Un conjugué avec une rhodamine a été obtenu comme suit :

### Rhodamine A

### 4-((3-((tert-butoxycarbonyl)amino)propyl)carbamoyl)-2-(6-(dimethylamino)-3-(dimethyliminio)-3H-xanthen-9-yl)benzoate

A une solution de 6-carboxytétraméthylrhodamine (156,1 mg, 362,6 µmol) dans le DMF sont ajoutés la DIPEA (120 µl ; 362,6 µmol) et le PyBOP (188,7 mg, 725 µmol). Apres 10 min, le N-Boc-1,3-propanediamine est ajouté (63,18 mg, 362,6 µmol) et le mélange réactionnel est agité pendant 4h à l'abri de la lumière. Apres évaporation du solvant, le brut réactionnel est purifié par HPLC, pour donner le produit attendu (40 mg, 68,2 µmol, 18,9% de rendement).

RMN 1 H (400MHz, Méthanol-d4): δ = 8,68 (s, 1 H), 8,18 (d, J = 8,0 Hz, 1 H), 7,47 (d, J = 8,0 Hz, 1 H), 7,21 (d, J = 9,5 Hz, 2 H), 7,05 (d, J = 9,5 Hz, 2 H), 6,97 (s, 2 H), 3,51 (t, J = 7,0 Hz, 2 H), 3,32 (s, 12 H), 3,19 (t, J = 7,0 Hz, 2 H), 1,88 - 1,79 (m, 2 H), 1,47 (s, 9 H)

SM (IES): [M+H]⁺ = 587,6

### Rhodamine B

### 4-((3-(5-(4-(((3-azidopropyl)((1-(4-carboxybutyl)-1H-1,2,3-triazol-4-yl)methyl)amino)methyl)-1H-1,2,3-triazol-1-yl)pentanamido)propyl)carbamoyl)-2-(6(dimethylamino)-3-(dimethyliminio)-3H-xanthen-9-yl)benzoate

A une solution de la rhodamine A (12,6 mg, 21,5 µmol) dans le CH₂Cl₂ distillé (500 µl) est ajouté le TFA (200 µl) à température ambiante, le mélange réactionnel est agité pendant 3h à l'abri de la lumière. Apres évaporation du solvant, le brut réactionnel est repris dans le DMF, puis la DIPEA (16 µl ; 96,8 µmol), l'acide 5,5'-(4,4'-(((3-azidopropyl)azanediyl) bis(methylene))bis(1H-1,2,3-triazole-4,1-diyl)) dipentanoique (14,9 mg, 32,3 µmol) et le PyBOP (16,8 mg, 32,3 µmol) sont ajoutés. Le mélange réactionnel est agité à température ambiante pendant 4h à l'abri de la lumière. Apres évaporation du solvant, le brut réactionnel est purifié par HPLC, pour donner le produit attendu (1,4 mg, 1,5 mmol, 7% de rendement).

RMN ¹H (400MHz, Méthanol-*d4*): δ = 8,74 (s, 1 H), 8,21 (d, *J* = 8,0 Hz, 1 H), 8,13 (s, 1 H), 8,09 (s, 1 H), 7,49 (d, *J =* 8,0 Hz, 1 H), 7,18 (d, *J* = 9,5 Hz, 2 H), 7,06 (d, *J* = 9,5 Hz, 2 H), 6,98 (s, 2 H), 4,52 - 4,40 (m, 6 H), 4,08 - 3,99 (m, 4 H), 3,54 - 3,46 (m, 4 H), 3,34 - 3,30 (m, 20 H), 2,32 - 2,20 (m, 4 H), 2,01 - 1,90 (m, 4 H), 1,88 - 1,81 (m, 1 H), 1,63 - 1,55 (m, 1 H)

SM (IES): [M+H]⁺ = 931,6

### Préparation 4 : 5-(4-(((3-azidopropyl)((1-(tert-butyl)-1H-1,2,3-triazol-4-yl)méthyl)amino)méthyl)-1H-1,2,3-triazol-1-yl) pentanoate d'éthyle

### Stade 1 : ethyl 5-(4-(((3-hydroxypropyl)(prop-2-yn-1-yl)amino)methyl)-1H-1,2,3-triazol-1-yl)pentanoate

A une solution de 3-(di(prop-2-yn-1-yl)amino)propan-1-ol 2 (1,32 g, 0,0873 mol) dans 5 mL d'eau / ^{t}BuOH sont ajoutés, le CuSO₄.5H₂O (207 mg, 8,7 mmol) et l'ascorbate de Sodium (1,72 g, 0,0873 mol) puis l'ethyl 5-azidopentanoate (1,49 g, 0,0873 mol) solubilisé dans 5 mL d'eau / ^{t}BuOH est ajouté sur 5h au pousse seringue. La réaction est soumise à agitation à température ambiante pendant 12h. Le brut réactionnel est évaporé à sec et purifié sur colonne de silice (EtOAc/MeOH, 90/10) pour obtenir le produit attendu (450 mg, 13,9 mmol, rendement de 16 %).

RMN ¹H (MeOD, 400 MHz) : δ 7,90 (s, 1 H), 4,42 (t, 2H, J = 8,0 Hz), 4,11 (q, 2H, J = 8,0 Hz), 3,82 (s, 2H), 3,61 (t, 2H, J = 8,0 Hz), 3,39 (s, 2H), 2,69-2,65 (m, 3H), 2,36 (t, 2H, J = 8,0 Hz), 1,94 (tt, 2H, J = 8,0 Hz), 1,73 (tt, 2H, J = 8,0, 8,0 Hz), 1,60 (tt, 2H, J = 8,0, 8,0 Hz), 1,24 (t, 3H, J = 8,0 Hz) ppm.

RMN ¹³C (CDCl3, 100 MHz) : δ 172,7, 144,2, 122,4, 77,6, 73,7, 62,9, 60,2, 53,3, 51,9, 49,7, 48,3, 41,7, 33,2, 29,4, 28,2, 28,1, 21,5, 14,0 ppm.

IR (Pastilles NaCl): v = 3291, 2948, 1725, 1462, 1440, 1122, 1064 cm⁻¹;

### Stade 2 : 5-(4-((((1-(tert-butyl)-1H-1,2,3-triazol-4-yl)méthyl)(3-hydroxypropyl) amino)méthyl)-1H-1,2,3-triazol-1-yl) pentanoate diéthyle

A une solution de 5-(4-(((3-hydroxypropyl)(prop-2-yn-1-yl)amino)methyl)-1H-1,2,3-triazol-1-yl) pentanoate d'éthyle (647 mg, 2,008 mmol) dans 5 mL d'eau / ^{t}BuOH sont ajoutés le 2-azido-2-methylpropane (596 g, 596 µL, 6,024 mmol), le CuSO₄.5H₂O (49,94 mg, 0,20 mmol) et l'ascorbate de Sodium (397,8 mg, 2,008 mol). La réaction est soumise à agitation à température ambiante pendant 12h. La réaction n'étant pas complète, les mêmes quantités de cuivre et d'ascorbate sont ajoutés en même temps que 2 équivalents de 2-azido-2-methylpropane. Le brut réactionnel est évaporé à sec et purifié sur colonne de silice (EtOAc/MeOH) pour obtenir le produit attendu (425 mg, rendement de 50 %).

RMN ¹H (400MHz *,Chloroforme-d*) δ = 7,72 (s, 1H), 7,70 (s, 1H), 4,34 (t, 2H, J = 8 Hz), 4,09 (q, 2H, J = 8 Hz), 3,77 (s, 2H), 3,75 (s, 2H), 3,73 (t, 2H, J = 8Hz), 2,76 (t, 2H, J = 8 Hz), 2,31 (t, 2H, J = 8 Hz), 1,81 (quint, 2H, J = 8 Hz), 1,71 (quint, 2H, J = 8 Hz), 1,64 (s, 9H), 1,21 (t, 3H, J = 8 Hz) ppm ;
RMN ¹³C (101MHz ,Méthanol-*d4*) δ = 175, 173, 145, 144, 126, 123, 117, 61,6, 61,1, 51,8, 51,1, 34,4, 30,7, 30,3 (3C), 23,0, 21,0, 14,6 ppm ;
IR (pastilles NaCl): 3412, 2940, 1729, 1612, 1372, 1197, 1051 cm⁻¹ ;

### Stade 3 : 5-(4-(((3-azidopropyl)((1-(tert-butyl)-1H-1,2,3-triazol-4-yl)methyl) amino)methyl)-1H-1,2,3-triazol-1-yl)pentanoate d'éthyle

A une solution de 5-(4-((((1-(tert-butyl)-1H-1,2,3-triazol-4-yl)methyl)(3-hydroxypropyl) amino)methyl)-1H-1,2,3-triazol-1-yl)pentanoate d'éthyle (411 mg, 0,976 mmol) dans du DMF anhydre (4 mL) sont ajoutés le TEA (198 mg, 1,952 mmol), le MsCl (112 mg, 0,976 mmol). Après 2h d'agitation sous atmosphère inerte, on ajoute le NaN₃ (95,2 mg, 1,464 mmol) et le mélange réactionnel est agité pendant 12h à température ambiante et sous N₂. Le brut réactionnel est concentré sous vide et le produit brut est purifié sur colonne de silice (EtOAc/MeOH) pour donner le produit pur attendu (130 mg, 30% de rendement).

RMN ¹H (400MHz ,Méthanol-*d4*) δ = 8,03 (s, 1 H), 7,97 (s, 1 H), 4,43 (t, 2H, J = 8 Hz), 4,09 (q, 2H, J = 8 Hz), 3,76 (s, 2H), 3,75 (s, 2H), 3,35 (t, 2H, J = 4 Hz), 2,52 (t, 2H, J = 8 Hz), 2,36 (t, 2H, J = 8 Hz), 1,95 (quint, 2H, J = 8 Hz), 1,81 (quint, 2H, J = 8 Hz), 1,68 (s, 9H), 1,60 (quint, 2H, J = 4 Hz), 1,21 (t, 3H, J = 8 Hz) ppm ;
IR (pastilles NaCl): 3417, 2940, 2097, 1730, 1459, 1373, 1208, 1047, 827 cm⁻¹

### Exemple 2 : Acide 5-(4-(((3-azidopropyl)((1-(tert-butyl)-1H-1,2,3-triazol-4-yl)méthyl)amino)méthyl)-1H-1,2,3-triazol-1-yl)pentanoique

A une solution de 5-(4-(((3-azidopropyl)((1-(tert-butyl)-1H-1,2,3-triazol-4-yl)méthyl)amino)méthyl)-1H-1,2,3-triazol-1-yl) pentanoate d'éthyle de la préparation 4 (94,5 mg, 0,2286 mmol) dans une solution de NaOH (2M, 17,73 mg), EtOH (300 µL) est ajouté pour rendre le milieu réactionnel homogène. La solution est agitée à température ambiante pendant 24h et l'éthanol est évaporé à sec. La phase aqueuse est acidifiée jusqu'à pH = 7 avec une solution de HCl (1 M) et évaporée. Le brut réactionnel est dissous dans du MeOH glacé et les sels sont filtrés 2 fois et on obtient le produit attendu (96 mg, rendement de 99 %).

RMN ¹H (400MHz ,Méthanol-*d4*) δ = 8,11 (s, 1 H), 8,06 (s, 1H), 4,45 (t, 2H, J = 8 Hz), 3,91 (s, 2H), 3,90 (s, 2H), 3,36 (t, 2H, J = 4 Hz), 2,66 (t, 2H, J = 4 Hz), 2,34 (t, 2H, J = 8 Hz), 1,97 (quint, 2H, J = 8 Hz), 1,86 (quint, 2H, J = 8 Hz), 1,68 (s, 9H), 1,60 (quint, 2H, J = 8 Hz) ppm ;
RMN ¹³C (101 MHz ,Méthanol-*d4*) δ = 177, 144, 143, 126, 123, 31,1, 51,2, 34,4, 30,8, 30,3 (3C), 27,1, 23,1 ppm ;
IR (pastilles NaCl): 3408, 2979, 2364, 2099, 1711, 1461, 1209, 1052, 823 cm⁻¹

### Préparation 5 : di-tert-butyl (((4,4'-(4,4'-(((3-azidopropyl)azanediyl) bis(methylene))bis(1H-1,2,3-triazole-4,1-diyl))bis(butanoyl)bis(azanediyl)) bis(propane-3,1-diyl))dicarbamate

### Stade 1 : di-tert-butyl (((4,4'-(4,4'-(((3-hydroxypropyl)azanediyl)bis(methylene)) bis(1H-1,2,3-triazole-4,1-diyl))bis(butanoyl))bis(azanediyl)) bis(propane-3,1-diyl))dicarbamate

A une solution de 3-(di(prop-2-yn-1-yl)amino)propan-1-ol **2** (731 mg, 4,8 mmol) dans 10 mL d'eau / tBuOH (1:1) sont ajoutés le *tert-*butyl (3-(4-azidobutanamido)propyl)carbamate (1,38 g, 4,8 mmol), le CuSO₄.5H₂O (120 mg, 0,48 mmol) et l'ascorbate de sodium (950 mg, 4,8 mmol). La réaction est soumise à agitation à température ambiante pendant 12h. Le brut réactionnel est évaporé à sec et purifié sur colonne de silice (CH₂Cl₂/MeOH 8 :2) pour obtenir le produit attendu (820 mg, rendement de 24 %).

RMN ¹H (400MHz, *Chloroforme-d*) δ = 7,74 (s, 2 H), 7,00 (br. s, 2 H), 5,14 (br. s, 2 H), 4,42 (t, *J* = 6,0 Hz, 4 H), 3,86 (s, 4 H), 3,68 (t, *J =* 5,0 Hz, 2 H), 3,29-3,27 (m, 4 H), 3,16-3,13 (m, 4 H), 2,93-2,90 (m, 2 H), 2,25-2,21 (m, 4 H), 2,17-2,14 (m, 4 H), 1,84-1,82 (m, 2 H), 1,65-1,63 (m, 4H), 1,41 (s, 18 H) ppm;
RMN ¹³C (101 MHz, *Chloroforme-d*): δ = 171,7 (2C), 156,4 (2C), 143,2 (2C), 124,1 (2C), 79,0 (2C), 62,4 (2C), 52,6, 49,3 (2C), 48,0 (2C), 37,3, 36,1, 32,2 (2C), 29,8 (2C), 28,3 (6C), 28,0 (2C), 25,8 (2C) ppm;
SM (IES): [M+H]⁺ = 722,6.

### Stade 2: di-tert-butyl (((4,4'-(4,4'-(((3-azidopropyl)azanediyl) bis(methylene))bis(1H-1,2,3-triazole-4,1-diyl)bis(butanoyl))bis(azanediyl)) bis(propane-3,1-diyl))dicarbamate

A une solution de di-*tert*-butyl (((4,4'-(4,4'-(((3-hydroxypropyl)azanediyl) bis(methylene)) bis(1H-1,2,3-triazole-4,1-diyl))bis(butanoyl))bis(azanediyl)) bis(propane-3,1-diyl))dicarbamate (530 mg, 0,706 mmol) du stade 1 dans du DMF anhydre (20 mL) sont ajoutés la TEA (296 µL, 2,12 mmol), et le MsCl (60 µL, 0,777 mmol). Après 2h d'agitation sous atmosphère inerte, l'azoture de sodium (230 mg, 3,53 mmol) est ajouté et le mélange réactionnel est agité pendant 12h à température ambiante sous N₂. Le brut réactionnel est concentré sous vide et le résidu est purifié sur colonne de silice (EtOAc/MeOH, 80:20) pour donner le produit pur attendu (270 mg, 0,348 mmol, 49% de rendement).

RMN ¹H (400MHz, *Chloroforme-d*): δ = 7,68 (s, 2 H), 6,83 (br. s, 2 H), 5,05 (br. s, 2 H), 4,42 (t, *J =* 6,5 Hz, 4 H), 3,76 (s, 4 H), 3,37 (t, *J =* 6,5 Hz, 2 H), 3,31-3,29 (m, 4 H), 3,16-3,14 (m, 4 H), 2,73-2,71 (m, 2 H), 2,26-2,24 (m, 4 H), 2,19-2,16 (m, 4 H), 1,91-1,88 (m, 2 H), 1,65-1,63 (m, 4H), 1,42 (s, 18 H) ppm;
RMN ¹³C (*101 MHz, Chloroforme-d*): δ = 171,7 (2C), 156,6 (2C), 148,5 (2C), 124,3 (2C), 79,4 (2C), 51,1, 49,5 (2C), 49,3 (2C), 47,8 (2C), 37,4, 36,2 (2C), 32,6 (2C), 30,2 (2C), 28,5 (6C), 26,5, 26,1 (2C) ppm.

### Exemple 3 : 4,4'-(4,4'-(((3-azidopropyl)azanediyl)bis(methylene))bis(1H-1,2,3-triazole-4,1-diyl))bis(N-(3-aminopropyl)butanamide)

A une solution de di-*tert*-butyl (((4,4'-(4,4'-(((3-azidopropyl)azanediyl) bis(methylene))bis(1H-1,2,3-triazole-4,1-diyl))bis(butanoyl))bis(azanediyl)) bis(propane-3,1-diyl))dicarbamate (200 mg, 0,268 mmol) de la préparation 5 dans 4mL de CH₂Cl₂ est ajouté 1 mL de TFA. Après 2h d'agitation à température ambiante le milieu est concentré pour conduire au produit désiré sous forme d'une huile incolore (208 mg, 0,268 mmol, quanti.).

RMN ¹H (400MHz, *Methanol-d*): δ = 8,16 (s, 2 H), 4,38 (s, 4 H), 4,35 (t, *J =* 6,5 Hz, 4 H), 3,32 (t, *J =* 6,5 Hz, 2 H), 3,14-3,09 (m, 6 H), 2,81 (t, *J =* 7,5 Hz, 4 H), 2,12-2,04 (m, 10 H), 1,71-1,68 (m, 4H), ppm;
RMN ¹³C (101 MHz, *Methanol-d*): δ = 175,3 (2C), 137,6 (2C), 128,7 (2C), 51,6, 49,8 (2C), 47,9 (2C), 38,5, 38,4 (2C), 37,1 (2C), 33,4 (2C), 28,7 (2C), 27,3 (2C), 25,0 ppm.

### Expérimentation 1 : Mesure de l'activité des azotures vis à vis de réaction « click » azoture/alcyne en milieu tamponé

On a étudié les cinétiques de réaction de couplage azoture/alcyne obtenues en utilisant des azotures de la présente invention ainsi que les cinétiques obtenues en utilisant des azotures et des composés chélatants du cuivre de l'art antérieur.

Les composés de l'art antérieur ont été les suivants :

### Exemple comparatif 1

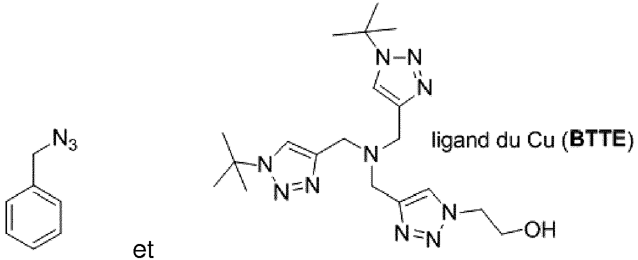

### Exemple comparatif 2

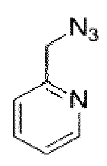

### Exemple comparatif 3

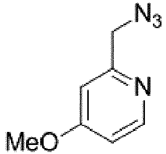

Les conditions opératoires étaient les suivantes :
Les cinétiques ont été réalisées dans des conditions de concentrations classiques en bio-conjugaison, c'est-à-dire en milieu aqueux à pH neutre et dans des conditions de basse concentration en réactifs. Azoture, alkyne et Cu 17,5 µM, Sodium ascorbate 475 µM (25 eq).

On a plus précisément procédé comme suit :
La réactivité des azotures de l'invention vis-à-vis de la réaction « click » azoture/alcyne a été évaluée par un test fluorescent (schéma 1) permettant d'établir les paramètres cinétiques de la réaction et de les comparer avec les solutions de la littérature. Ce test utilise un alcyne pro-fluorescent C1 qui forme un produit de cycloaddition C2 hautement fluorescent. La synthèse et les propriétés de fluorescence sont décrites dans la littérature. 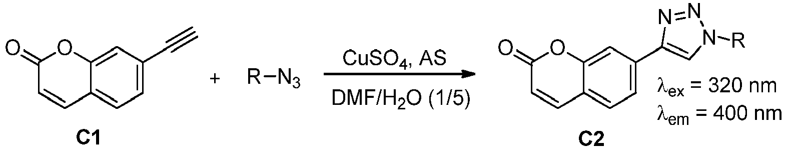
Schéma 1. Test fluorescent permettant d'évaluer la cinétique de la réaction de ligation.

Les études cinétiques ont été réalisées dans les conditions suivantes :
A 50 µL d'une solution aqueuse contenant 70 µM de CuSO₄ sont additionnés 100 µL d'une solution contenant 35 µM d'alcyne C1 et 35 µM d'azoture RN₃ dissous dans un mélange DMF/H₂O (50/50). La réaction est alors initiée par addition de 50 µL d'une solution aqueuse contenant 1,75 mM d'acide ascorbique (AS). La réaction de cycloaddition est suivie par mesure toutes les 5 secondes pendant 20 minutes de l'intensité de fluorescence à 400 nm suite à une excitation à 320 nm. Le rendement de la réaction, est calculé grâce à une gamme d'étalonnage préalablement établie avec le cyclo-adduit C2.

Les résultats obtenus sont les suivants :

| | Composé testé | Vitesse de réaction (µM/min) | Rendement de ligation |
|---|---|---|---|
| Exemple comparatif 1 | | 5,2 | 33% |
| Exemple comparatif 2 | | 4,2 | 28% |
| Exemple comparatif 3 | | 3,5 | 35% |
| Exemple de référence | Préparation 1 | 101,7 | 65% |
| Exemple de référence | Préparation 2 | 10,8 | 45% |
| Exemple de référence | Préparation 3 | 16,5 | 67% |
| Exemple | Exemple 1 | 12,0 | 74% |

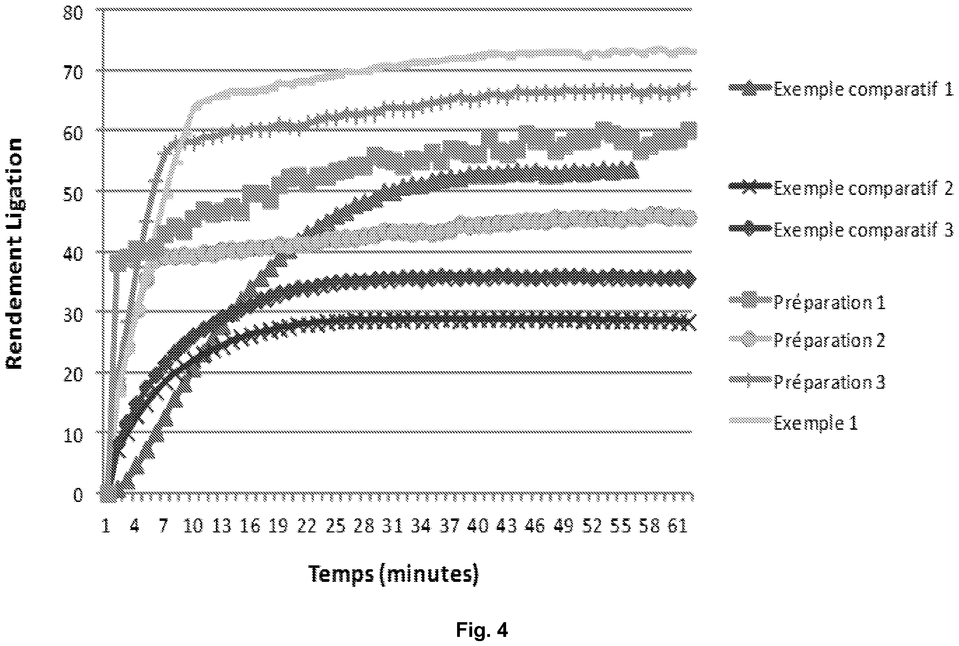

Les cinétiques sont représentées sur la figure 4.

Les vitesses de réactions ainsi que les rendements de ligation sont globalement supérieurs pour les azotures de la présente invention en comparaison de ceux des composés comparables de l'art antérieur. L'azoture de la préparation 1 par exemple permet une réaction « click » environ 20 fois plus rapide, et l'azoture de la préparation 3 ou de l'exemple 1 des rendements deux fois supérieurs à ceux des meilleurs composés comparables de l'art antérieur.

### Expérimentation 2 : cinétique de ligation en milieu complexe

On a également procédé à une expérimentation dans un milieu de culture cellulaire. On a opéré comme suit :
Les études cinétiques ont été réalisées dans les conditions suivantes :
   A 50 µL de lysat cellulaire contenant 1,4 mM de CuSO₄ sont additionnés 100 µL d'une solution contenant 35 µM d'alcyne C1 et 35 µM d'azoture RN₃ dissous dans du lysat cellulaire. La réaction est alors initiée par addition de 50 µL d'une solution aqueuse contenant 1,75 mM d'acide ascorbique (AS). La réaction de cycloaddition est suivie par mesure toutes les 5 secondes pendant 20 minutes de l'intensité de fluorescence à 400 nm suite à une excitation à 320 nm. Le rendement de la réaction, est calculé grâce à une gamme d'étalonnage préalablement établie avec le cyclo-adduit C2.

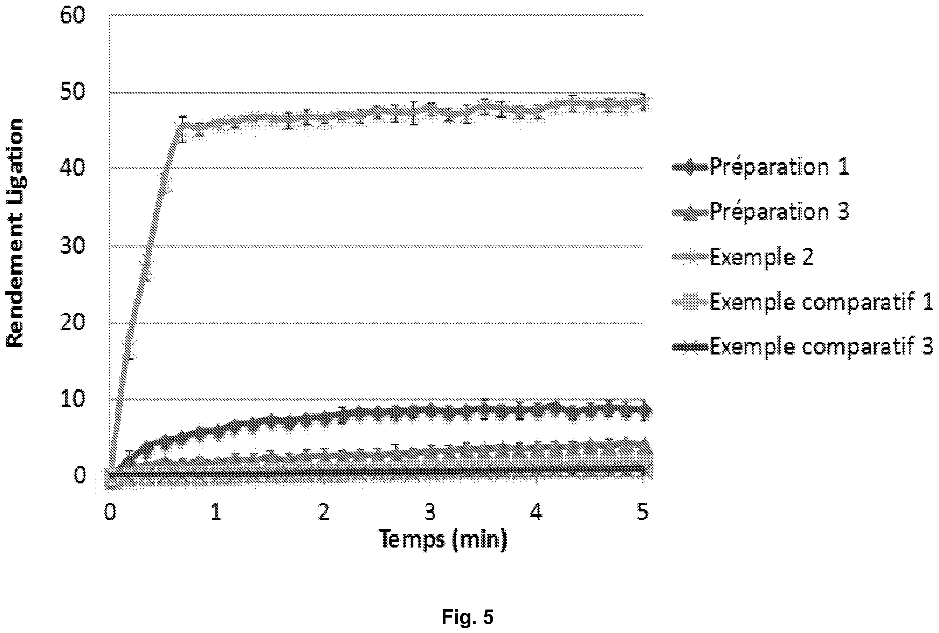

Les résultats obtenus sont représentés sur la figure 5 qui montre donc les cinétiques comparatives en milieu complexe (lysat cellulaire) de formation du produit C2 en fonction des azotures employés. Conditions: Azoture et alkyne 17,5 µM, Cu 350 µM, Sodium ascorbate 8,75 mM (500 eq).

La courbe supérieure correspond à l'azoture de l'exemple 2, les courbes inférieures aux exemples comparatif 1 et 3 et la courbe du milieu à la préparation 3

L'examen de la figure montre que la différence de réactivité entre les azotures de la présente invention et les composés de l'art antérieur est encore plus prononcée lorsque la réaction « click » est effectuée en milieu complexe, tel qu'un milieu de lysat cellulaire.

### Expérimentation 3 : Cinétique de ligation en milieu simple et complexe

Les groupements F1 et F2 des composés de la présente invention ont une double fonctionnalité, comme on va le montrer ci-après. En effet,
i) ils permettent le couplage de l'azoture chélatant via un groupement fonctionnel situé sur F1 et/ou sur F2 et
ii) ils modulent la réactivité de l'azoture chélatant vis-à-vis de la réaction click.

Les cinétiques réactionnelles ont été déterminées par un test fluorescent illustré par la réaction ci-dessous, permettant d'établir les paramètres cinétiques de la réaction et de les comparer avec les solutions de la littérature. Ce test utilise un alcyne pro-fluorescent qui forme un produit de cycloaddition hautement fluorescent.

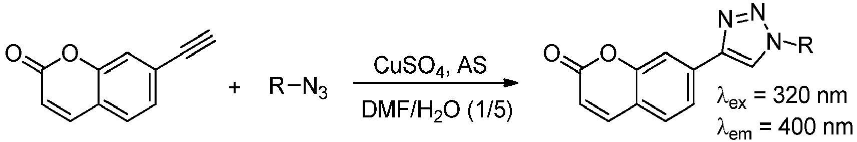

On a testé le composé nommé A14 qui est un azoture de structure similaire à ceux décrits dans les articles de GILLES GASSER ET AL. "Synthesis, characterisation and bioimaging of a fluorescent rhénium-containing PNA bioconjugate", DALTON TRANSACTIONS, vol 41, no. 8, 20-12-2011, pp. 2304-2313 et GASSER ET AL. "Preparation 99m Tc labeling and biodistribution studies of a PNA oligomer containing a new ligand derivate of 2,2-dipicolylamine", JOURNAL OF INORGANIC CHEMISTRY, vol 104, no. 11, 31 Juillet 2010, pp. 1133-1140 ainsi que les composés des exemples 1 et 2 de la présente invention, nommés respectivement A4 et A20

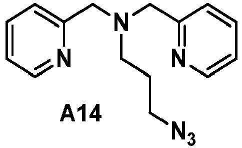

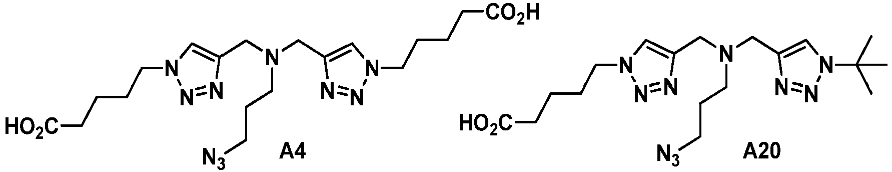

Les conditions opératoires sont les suivantes : on a utilisé la même concentration 17 µM pour l'alcyne et pour l'azoture. La concentration de sulfate de cuivre a été de 34 µM et celle d'ascorbate de sodium (AS) de 850 µM.

On a opéré d'une part dans un tampon phosphate 0.1 M à pH 7,4 et d'autre part dans un lysat cellulaire obtenu par 3 cycles de sonication de 30 secondes de cellules Jurkat (myélome humain). L'alcyne, l'azoture et le sulfate de cuivre sont mélangés à température ambiante pendant 30 minutes puis la cinétique est démarrée par addition d'ascorbate de sodium.

Les résultats des cinétiques sont montrés sur les figures 6 et 7. Comme le montrent les figures 6 et 7, le composé A14 est inactif dans les conditions utilisées que ce soit en milieu complexe comme un lysat cellulaire ou un milieu très simple comme le tampon phosphate. Seuls les azotures chélatants de l'invention (A4, A20) permettent de réaliser la réaction click avec efficacité. L'influence des groupements F1 et F2 sur l'efficacité de la réaction est également démontrée dans les résultats illustrés par la figure 1. Une différence de réactivité est déjà observée entre les composés des exemples 1 et 2 dans le milieu tampon phosphate. Cette différence devient plus que significative dans un milieu complexe comme celui d'un lysat cellulaire. Dans ces conditions le composé de l'exemple 2 est particulièrement efficace.

### Expérimentation 4: Ligation dans les cellules vivantes

La remarquable efficacité des composés azotures chélatants de l'invention a été confirmée par des expériences menées sur des cellules vivantes. Les résultats sont illustrés sur la figure 8.

On a tout d'abord préparé le composé fluorescent 1 dont la structure est montrée ci-dessous, 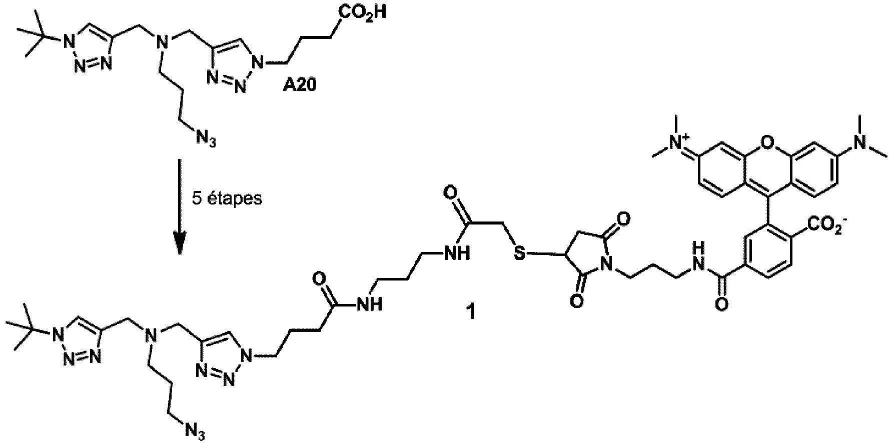 à partir du composé de l'exemple 2 en cinq étapes, en utilisant sa fonction acide carboxylique, en procédant comme suit :

### Stade A: tert-butyl (3-(4-(4-(((3-azidopropyl)((1-(tert-butyl)-1 H-1,2,3-triazol-4-yl)methyl)amino)methyl)-1H-1,2,3-triazol-1-yl)butanamido)propyl)carbamate

A une solution de composé de l'exemple 2 (A20) (213 mg, 0.49 mmol) dans 5 ml de THF, sont additionnés un équivalent de NHS et de la DCC. La réaction est agitée à température ambiante pendant 1 h. Le N-Boc-1,3-propanediamine (0.297 g, 1.7 mmol) dissous dans 5 ml de THF est additionné à la solution qui est agitée une nuit à température ambiante. Après évaporation du solvant, le résidu est purifié sur gel de silice pour obtenir 118 mg (0.22 mmol, rendement 45%) du produit attendu.

¹H NMR (400MHz, CHLOROFORM-d) δ = 7.66 (s, 1 H), 7.63 (s, 1 H), 6.85 (broad s, 1 H), 5.04 (broad s, 1 H), 4.42 (t, *J* = 6.0 Hz, 2 H), 3.74 (s, 2 H), 3.67 (s, 2 H), 3.37-3.31 (m, 4 H), 3.16 (dt, *J* = 6.0 Hz, *J* = 6.0 Hz, 2 H), 2.63 (t, *J* = 6.5 Hz, 2 H), 2.27-2.15 (m, 4 H), 1.86 (tt, *J* = 6.5 Hz, *J* = 6.5 Hz, 2 H), 1.67-1.62 (m, 11 H), 1.42 (s, 9 H);
¹³C NMR (101 MHz, CHLOROFORM-d) δ = 171.7, 156.4, 143.9, 143.6, 123.9, 120.2, 79.2, 77.2, 59.3, 50.5, 49.3, 49.1, 47.8, 47.5, 32.5, 30.1, 30.0, 29.6, 28.4, 26.6, 26.1;
IR (NaCl, cm⁻¹): 3375, 2978, 2940, 2098, 1689, 1652, 1533, 1454, 1368, 1276, 1252, 1170, 1051;
MS (ESI) *m*/*z*: 562 [M+H]⁺.

### Stade B: N-(3-aminopropyl)-4-(((3-azidopropyl)((1-(tert-butyl)-1H-1,2,3-triazol-4-yl)methyl)amino)methyl)-1H-1,2,3-triazol-1-yl)butanamide

Tert-butyl(3-(4-(4-(((3-azidopropyl)((1-(tert-butyl)-1H-1,2,3-triazol-4-yl)methyl)amino)methyl)-1H-1,2,3-triazol-1-yl)butanamido)propyl)carbamate (95 mg, 0.17 mmol) est dissous dans 0.5 mL de dichloromethane puis 200 µL de TFA sont additionnés. Après 2 h de réaction, le solvant est évaporé et le produit attend est obtenu sous l'aspect d'une huile incolore (97.6 mg, 0.17 mmol, rdt 100%).

¹H NMR (400MHz, METHANOL-d₄) δ = 8.35 (s, 1 H), 8.32 (s, 1 H), 4.54-4.52 (m, 6 H), 3.49 (t, *J* = 6.5 Hz, 2 H), 3.29-3.22 (m, 4 H), 2.96 (t, *J* = 7.0 Hz, 2 H), 2.30-2.21 (m, 4 H), 2.18-2.11 (m, 2 H), 1.84 (tt, *J* = 7.0 Hz, *J* = 7.0 Hz, 2 H), 1.71 (s, 9 H);
¹³C NMR (101 MHz, METHANOL-d₄) δ = 175.2, 137.4, 137.0, 128.5, 125.6, 61.6, 51.5, 49.5, 48.1, 47.9, 38.3, 37.0, 33.2, 30.1, 28.7, 27.1;
IR (NaCl, cm⁻¹): 3405, 2985, 2929, 2105, 1678, 1646, 1553, 1466, 1428, 1374, 1267, 1202, 1131, 1055, 1026, 835, 800, 723, 474;
MS (ESI) *mlz*: 461 [M+H]⁺.

HRMS (ESI): calculated for C₂₀H₃₇N₁₂O⁺ [M+H]⁺: 461.3213, found: 461.3220.

### Stade C: S-(2-((3-(4-(4-(((3-azidopropyl)((1-(tert-butyl)-1H-1,2,3-triazol-4-yl)methyl)amino)methyl)-1H-1,2,3-triazol-1-yl)butanamido)propyl)amino)-2-oxoethyl)ethanethioate

Du N-(3-aminopropyl)-4-(((3-azidopropyl)((1-(tert-butyl)-1H-1,2,3-triazol-4-yl)methyl)amino)methyl)-1H-1,2,3-triazol-1-yl)butanamide (71.4 mg, 124 µmol) est dissous dans 1.5 mL de DMF et de la TEA est additionnée (21 µl, 149 µmol), la réaction est agitée à température ambiante penadant 10 min. Du 2,5-dioxopyrrolidin-1-yl-2-(acetylthio)acetate est additionné et la réaction est agitée pendant 5h. Le solvant est évaporé puis le résidu est purifié sur colonne de silice (CH₂Cl₂/MeOH: 90/10) pour conduire au produit attendu (38 mg, 66 µmol, yield 53%).

¹H NMR (400MHz, CHLOROFORM-d) δ = 7.67 (s, 1 H), 7.63 (s, 1 H), 6.94 (broad s, 2 H), 4.42 (t, *J* = 6.0 Hz, 2 H), 3.74 (s, 2 H), 3.66 (s, 2 H), 3.55 (s, 2 H), 3.36 (t, *J* = 6.5 Hz, 2 H), 3.32-3.21 (m, 4 H), 2.62 (t, *J* = 6.5 Hz, 2 H), 2.40 (s, 3 H), 2.28-2.13 (m, 4 H), 1.86 (tt, *J* = 6.5 Hz, *J* = 6.5 Hz, 2 H), 1.66 (s, 9 H);
¹³C NMR (101 MHz, CHLOROFORM-d) δ = 195.5, 172.0, 168.4, 144.0, 143.6, 124.0, 120.2, 59.4, 50.5, 49.3, 49.1, 47.8, 47.4, 36.4, 36.0, 33.1, 32.5, 30.3, 30.0, 29.4, 26.6, 26.1;
IR (NaCl, cm⁻¹): 3413, 2097, 1646, 1545, 1440, 1372, 1210, 1133, 1049,960,489,459;
MS (ESI) *mlz*: 577 [M+H]⁺.

### Stade D: 4-((3-(3((2-((3-(4-(4-(((3-azidopropyl)((1-(tert-butyl)-1H-1,2,3-triazol-4-yl)methyl)amino)methyl)-1H-1,2,3-triazol-1-yl)butanamido)propyl)amino)-2-oxoethyl)thio)-2,5-dioxopyrrolidin-1-yl)propyl)carbamoyl)-2-(6-(dimethylamino)-3-(dimethyliminio)-3H-xanthen-9-yl)-benzoate

Du S-(2-((3-(4-(4-(((3-azidopropyl)((1-(tert-butyl)-1H-1,2,3-triazol-4-yl)methyl)amino)methyl)-1H-1,2,3-triazol-1-yl)butanamido)propyl)amino)-2-oxoethyl)ethanethioate (16.48 mg, 28.6 µmol) est dissous dans 240 µl de EtOH et NaOH 1 N est additionné (95 µl, 95 µmol), la réaction est agitée à température ambiante pendant 3h. Du tétraméthylrhodamine (TAMRA)-maleimide (16.19 mg, 28.6 µmol) dissous dans 1.5 mL de EtOH est additionné et la réaction est agitée pendant 1h30. Le solvant est évaporé et le résidu purifié par HPLC-préparative, pour conduire au produit désiré (4.7 mg, 4.3 µmol, yield 15%).

¹H NMR (400MHz, CHLOROFORM-d) δ = 8.81 (s, 1 H), 8.27 (dd, *J* = 2.0 Hz, *J* = 8.0 Hz, 1 H), 8.19-8.15 (m, 1 H), 8.14-8.05 (m, 2 H), 8.02 (s, 1 H), 7.99 (s, 1 H), 7.89-7.82 (broad s, 1 H), 7.15 (d, *J* = 9.5 Hz, 1 H), 7.14 (d, *J =* 9.5 Hz, 1 H), 6.81 (dd, *J* =2.0 Hz, *J* = 9.5 Hz, 2 H), 6.72 (d, *J* = 2.0 Hz, 2 H), 4.35-4.25 (m, 4 H), 4.05-3.97 (m, 5 H), 3.91 (d, *J =* 15.0 Hz, 1 H), 3.59-3.44 (m, 3 H), 3.36-3.30 (m, 2 H), 3.29-3.22 (m, 14 H), 3.22-3.16 (m, 3 H), 2.84 (t, *J* = 7.5 Hz, 2 H), 2.57 (dd, *J* = 3.5 Hz, *J =* 19.0 Hz, 1 H), 2.15-2.06 (m, 2 H), 2.06-1.95 (m, 6 H), 1.67 (s, 9 H);
¹³C NMR (101MHz, METHANOL-d₄) δ = 178.5, 177.0, 174.4, 171.4, 168.5, 161.2, 159.0, 158.9, 137.7, 137.5, 132.2, 131.5, 131.2, 130.7, 115.4, 114.8, 97.4, 50.0, 49.8, 49.6, 49.5, 49.3, 49.1, 41.1, 40.9, 38.5, 38.1, 37.6, 37.7, 36.7, 35.6, 33.5, 30.1, 30.0, 28.4, 27.3, 26.5;
MS (ESI) *m*/*z*: 1101 [M+H]⁺.

On a ensuite préparé le complexe au cuivre de ce composé fluorescent 1 comme suit :
Le composé fluorescent 1 est mélangé en présence d'un équivalent de sulfate de cuivre dissous dans l'eau. Le mélange est agité pendant 1 h à température ambiante puis 50 équivalents d'ascorbate de sodium sont additionnés, la réaction est agitée pendant 30 minutes pour former quantitativement le complexe de cuivre(I).

Des cellules HuH-7 fixées sur des plaques de 96 puits, préalablement incubées en présence de paclitaxel 2 comportant une fonction alcyne ont été traitées par le complexe au cuivre ci-dessus.

Les cellules ont été traitées par 100 µL d'une solution 62.5 nM du composé 2 dissous dans du tampon phosphate 0.1M puis incubées à 37°C pendant 30 min. Les cellules sont alors lavées deux fois par addition de 100 µL de tampon phosphate 0.1 M puis traitées pendant 4 h par 100 µL d'une solution 100µM du complexe de cuivre du composé 1. Les cellules sont à nouveau lavées deux fois par addition de 100 µL de tampon phosphate 0.1 M puis fixées par traitement de 150 µL d'une solution à 4% de formaldéhyde. Après fixation pendant 15 min, les cellules sont lavées deux fois par addition de 100 µL de tampon phosphate 0.1M puis incubées par une solution 62.5 nM de Tubulin Tracker® Green 3 pendant 30 min à 37°C. Les cellules sont à nouveau lavées par du tampon phosphate 0.1M puis observées par microscopie par fluorescence.

Après des étapes de lavages des cellules, la fluorescence de la partie rhodamine du complexe au cuivre du composé 1 a été mesurée. La figure 8B montre la fluorescence des cellules incubées avec le paclitaxel-alcyne 2 puis traitées par l'azoture chélatant 1 en présence de cuivre. Le composé 3, conjugué covalent entre le paclitaxel et le fluorophore Oregon Green®, a été utilisé comme témoin.

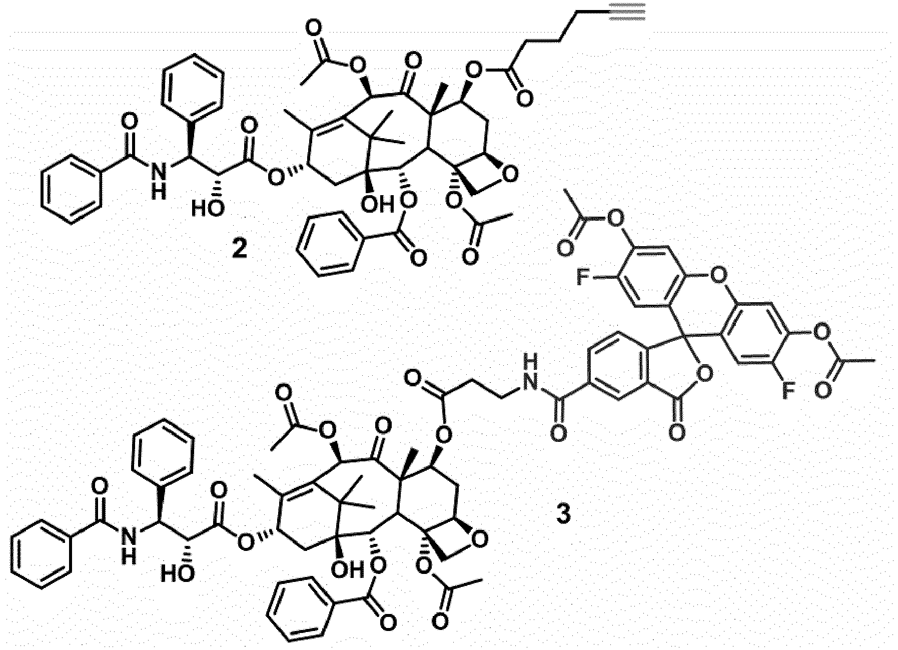

La figure 8A illustre la localisation du noyau cellulaire par le réactif 4',6'-diamidino-2-phénylindole. La figure 8C montre la fluorescence verte mesurée après incubation du composé 3 sur les mêmes cellules incubées et sert de témoin et enfin la figure 8D montre la co-localisation, par superposition des mesures de fluorescence de 1 et 3.

Les dérivés du paclitaxel sont bien connus pour se lier avec de très fortes affinités sur la tubuline des cellules. Les expériences illustrées par la figure 8 montrent que l'azoture 1 préparé à partir du composé de l'exemple 2, après avoir été complexé au cuivre, est capable de pénétrer à l'intérieur des cellules et de se coupler via une réaction « click » au paclitaxel-alcyne 2 lui-même fixé sur la tubuline comme montré sur la figure B par une fluorescence rouge.

La figure 8D, qui correspond à une superposition d'images de fluorescence de la rhodamine rouge liée au composé 1 et de l'oregon green® vers lié au composé 3, montre sur une image couleur la co-localisation des molécules (rouge + vert = jaune).

## Revendications

1. Un azoture de formule I 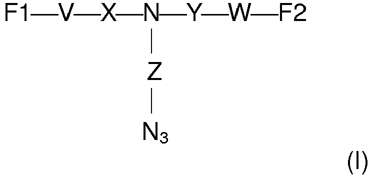 dans laquelle :
V et W représentent indépendamment l'un de l'autre un hétérocycle comprenant un hétéroatome complexant le cuivre, azoté, ou soufré, ledit hétérocycle étant saturé ou insaturé,
X et Y liés à l'azote central représentent indépendamment l'un de l'autre un groupement espaceur de structure -(CH₂)₂-O-(CH₂)-, - (CH₂)-O-(CH₂)₂-, -(CH₂)-O-(CH₂)-, -(CH₂)₂-S-(CH₂)-, -(CH₂)-S-(CH₂)₂-, -(CH₂)-S-(CH₂)-, -(CH₂)₂-NH-(CH₂)-, -(CH₂)-NH-(CH₂)₂--(CH₂)-NH-(CH₂)-, ou - (CH₂)ₘ-, où m peut prendre les valeurs 1, 2, 3,4;
Z représente un groupement espaceur de structure -(CH₂)₂-O-(CH₂)-, -(CH₂)-O-(CH₂)₂-, -(CH₂)₂-S-(CH₂)-, -(CH₂)-S-(CH₂)₂-, -(CH₂)₂-NH-(CH₂)--(CH₂)-NH-(CH₂)₂- ou, - (CH₂)ₙ -, où n peut prendre les valeurs 1, 2, 3,4;
F1 et F2 représentent indépendamment l'un de l'autre un atome d'hydrogène, ou un groupement alkyle tertiaire ayant au plus 7 atomes de carbone, ou un groupement fonctionnel permettant le couplage de l'azoture de formule I à une molécule chimique, à une biomolécule, à une nanoparticule ou à un polymère, ledit groupement fonctionnel étant un groupement isocyanate, isothiocyanate, carboxyl, amino, maléimide ou thiol, greffé indirectement sur les hétérocycles, étant entendu que au moins l'un de F1 et F2 représente un groupement fonctionnel.

2. Un azoture de formule I selon la revendication 1, **caractérisé en ce que** X représente un groupement - (CH₂)ₘ-, où m peut prendre les valeurs 1,2,3,4;

3. Un azoture de formule I selon la revendication 1 ou 2, **caractérisé en ce que** Y représente un groupement - (CH₂)ₘ-, où m peut prendre les valeurs 1, 2, 3, 4.

4. Un azoture de formule I selon l'une des revendications 1 à 3, **caractérisé en ce que** Z représente un groupement éthylène, propylène ou butylène.

5. Un azoture de formule I selon l'une des revendications 1 à 4, **caractérisé en ce que** V et W représentent indépendamment l'un de l'autre un hétérocycle azoté.

6. Un azoture de formule I selon l'une des revendications 1 à 4, **caractérisé en ce que** l'un au moins de F1 et F2 représente indépendamment de l'autre un groupement alkyle tertiaire ayant au plus 7 atomes de carbone.

7. Un azoture de formule I selon la revendication 6, **caractérisé en ce que** F1 et F2 représentent chacun indépendamment de l'autre un groupement *tert*-butyle.

8. Un azoture de formule I selon la revendication 1, choisi parmi
- l'acide 5,5'-(4,4'-(((3-azidopropyl)azanediyl) bis(methylene)) bis(1H-1,2,3-triazole-4,1-diyl)) dipentanoique,
- l'acide 5-(4-(((3-azidopropyl)((1-(tert-butyl)-1H-1,2,3-triazol-4-yl)méthyl)amino)méthyl)-1H-1,2,3-triazol-1-yl) pentanoique et
- le 4,4'-(4,4'-(((3-azidopropyl)azanediyl)bis(methylene))bis(1H-1,2,3-triazole-4,1-diyl))bis(N-(3-aminopropyl)butanamide).

9. Procédé de préparation d'un azoture de formule I tel que défini à l'une des revendications 1 à 8, dans laquelle X et Y représentent indépendamment l'un de l'autre un groupement - (CH₂)ₘ -, où m a la valeur 1, 2, 3 ou 4, **caractérisé en ce que** l'on fait réagir un amino alcool de formule II
OH-Z-NH₂ (II)
dans laquelle Z à la signification déjà indiquée, avec un aldéhyde de formule III
GP₁-F₁-V-X'-CHO (III)
dans laquelle F₁ et V ont la signification déjà indiquée, GP₁ représente un groupement protecteur d'une fonction F1 et X' représente un groupement - (CH₂)ₘ₋₁ - où m a la valeur déjà indiquée, pour obtenir un composé de formule IV
GP₁-F₁-V-X'-CH₂-NH-Z-OH (IV)
dans laquelle GP₁, F₁, V, X' et Z ont la signification déjà indiquée,
**que** l'on soumet, avec un aldéhyde de formule V
GP₂-F₂-W-Y'-CHO (V)
dans laquelle F₂ et W ont la signification déjà indiquée, GP₂ représente un groupement protecteur d'une fonction F2 et Y' représente un groupement - (CH₂)ₘ₋₁ - où m a la valeur déjà indiquée, à une amination réductrice pour obtenir un composé de formule VI 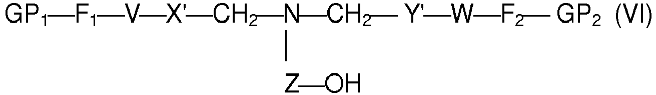 dans laquelle GP₁, F₁, V, X', Y', W, F₂, GP₂ et Z ont la signification déjà indiquée, que l'on fait réagir avec un azoture alcalin de formule VII
EN₃ (VII)
dans laquelle E représente un métal alcalin, pour obtenir un composé de formule VIII 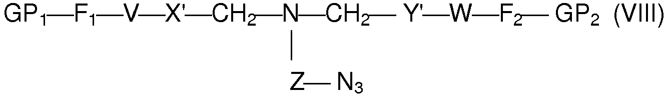 dans laquelle GP₁, F₁, V, X', Y', W, F₂, GP₂ et Z ont la signification déjà indiquée, dont on élimine les groupement protecteurs pour obtenir le composé de formule IA 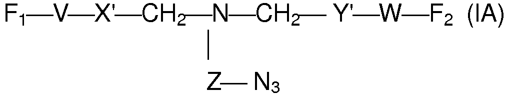 attendu que l'on isole si désiré.

10. Procédé de préparation d'un azoture de formule I tel que défini à l'une des revendications 1 à 8, dans laquelle V et W représentent un triazole, **caractérisé en ce que** l'on fait réagir un alcyne halogéné de formule Xa 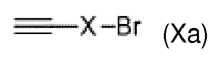 dans laquelle X à la signification déjà indiquée, et Br représente un atome d'halogène, de préférence un atome de brome, avec un amino alcool de formule XI
OH-Z-NH₂ (XI)
dans laquelle Z à la signification déjà indiquée, pour obtenir un dialcyne de formule XII 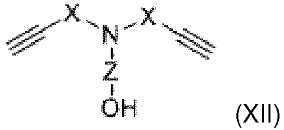 dans laquelle X et Z ont la signification déjà indiquée, que l'on fait réagir avec un azoture de formule XIII
GP₁-F₁-N₃ (XIII)
dans laquelle GP₁ et F₁ ont la signification déjà indiquée, en présence d'un catalyseur de préférence à base de cuivre, pour obtenir par cycloaddition un alcyne de formule XIV, 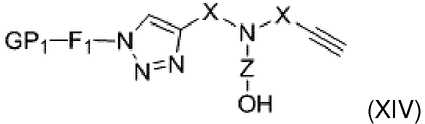 dans laquelle GP₁, F₁, X et Z ont la signification déjà indiquée, que l'on fait réagir avec un azoture de formule XV
GP₂-F₂-N₃ (XV)
dans laquelle GP₂ et F₂ ont la signification déjà indiquée, en présence d'un catalyseur de préférence à base de cuivre, pour obtenir par cycloaddition un composé de formule XVI, 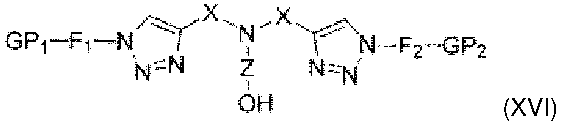 dans laquelle GP₁, GP₂, F₁, F₂, X et Z ont la signification déjà indiquée, que l'on fait réagir avec un azoture alcalin de formule VII
EN₃ (VII)
dans laquelle E a la signification déjà indiquée, pour obtenir un composé de formule XVII 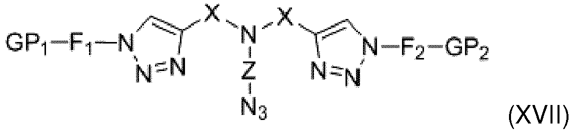 dans laquelle GP₁, GP₂, F₁, F₂, X et Z ont la signification déjà indiquée, dont on élimine les groupement protecteur pour obtenir le composé de formule IB 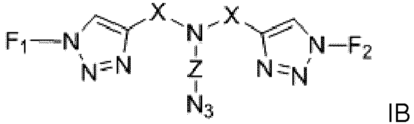 attendu dans laquelle F₁, F₂, X et Z ont la signification déjà indiquée, que l'on isole si désiré.

11. Utilisation d'un azoture de formule I tel que défini à l'une des revendications 1 à 8 pour la préparation de bioconjugués ou de nanoconjugués.

12. Utilisation d'un azoture de formule I tel que défini à l'une des revendications 1 à 8 pour l'isolation ou l'identification d'une cible protéique.

13. Utilisation d'un azoture de formule I tel que défini à l'une des revendications 1 à 8 dans l'imagerie.

14. Utilisation selon la revendication 12 pour l'isolation ou l'identification d'une cible protéique au sein d'une cellule.

## Patentansprüche

1. Azid mit der Formel I 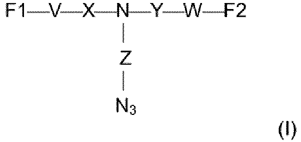 wobei:
V und W unabhängig voneinander für einen Heterozyklus stehen, umfassend ein Heteroatom, welches mit Kupfer einen Komplex bildet und stickstoff- oder schwefelhaltig ist, wobei der Heterozyklus gesättigt oder ungesättigt ist,
X und Y an den zentralen Stickstoff gebunden sind und unabhängig voneinander eine Spacergruppe mit folgender Struktur bilden -(CH₂)₂-O-(CH₂)-, -(CH₂) -O-(CH₂)₂-, -(CH₂)-O-(CH₂) -, -(CH₂)₂-S-(CH₂) -, -(CH₂) -S-(CH₂)₂ -, -(CH₂)-S-(CH₂) -, -(CH₂)₂ -NH-(CH₂) -, - (CH₂) -NH-(CH₂)₂ -, -(CH₂) -NH-(CH₂) -, oder (CH₂)ₘ -, wobei m die Werte 1, 2, 3, 4 haben kann;
Z für eine Spacergruppe mit folgender Struktur steht -(CH₂)₂-O-(CH₂) -, -(CH₂)-O-(CH₂)₂-, -(CH₂)₂-S-(CH₂) -, -(CH₂) -S-(CH₂)₂ -, -(CH₂)₂-NH-(CH₂) -, -(CH₂) -NH-(CH₂)₂ -, oder, (CH₂)ₙ-, wobei n die Werte 1, 2, 3, 4 haben kann;
F1 und F2 unabhängig voneinander für ein Wasserstoffatom stehen, oder eine tertiäre Alkylgruppe mit höchstens 7 Kohlenstoffatomen, oder eine funktionelle Gruppe welche eine Verknüpfung des Azids der Formel I mit einem chemischen Molekül, einem Biomolekül, einem Nanopartikel oder einem Polymer erlaubt, wobei die funktionelle Gruppe eine indirekt auf die Heterozyklen aufgepfropfte Isocyanat-, Isothiocyanat-, Carboxyl-, Amino-, Maleimid- oder Thiolgruppe ist, wobei wenigstens eines von F1 und F2 für eine funktionelle Gruppe steht.

2. Azid mit der Formel I nach Anspruch 1, **dadurch gekennzeichnet, dass** X für eine -(CH₂)ₘ- Gruppe steht, wobei m die Werte 1, 2, 3, 4 haben kann;

3. Azid mit der Formel I nach Anspruch 1 oder 2, **dadurch gekennzeichnet, dass** Y für eine -(CH₂)ₘ- Gruppe steht, wobei m die Werte 1, 2, 3, 4 haben kann;

4. Azid mit der Formel I nach einem der Ansprüche 1 bis 3,
**dadurch gekennzeichnet, dass** Z für eine Ethylen-, Propylen- oder Butylengruppe steht.

5. Azid mit der Formel I nach einem der Ansprüche 1 bis 4,
**dadurch gekennzeichnet, dass** V und W unabhängig voneinander für einen stickstoffhaltigen Heterozyklus stehen.

6. Azid mit der Formel I nach einem der Ansprüche 1 bis 4,
**dadurch gekennzeichnet, dass** wenigstens eines von F1 und F2 unabhängig voneinander für eine tertiäre Alkylgruppe mit höchstens 7 Kohlenstoffatomen steht.

7. Azid mit der Formel I nach Anspruch 6, **dadurch gekennzeichnet, dass** F1 und F2 unabhängig voneinander für eine tert-Butyl-Gruppe stehen.

8. Azid mit der Formel I nach Anspruch 1, gewählt aus
- 5,5'-(4,4'-(((3-azidopropyl)azanediyl)bis(methylen))bis(1H-1,2,3-triazol-4,1-diyl))dipentan-Säure,
- 5-(4-(((3-azidopropyl)((1-(tert-butyl)-1H-1,2,3-triazol-4-yl)methyl)amino)methyl)-1H-1,2,3-triazol-1-yl)pentan-Säure, und
- 4,4'-(4,4'-(((3-azidopropyl)azanediyl)bis(methylen))bis(1H-1,2,3-triazol-4,1-diyl))bis(N-(3-aminopropyl)butanamid).

9. Herstellungsverfahren für ein Azid mit der Formel I gemäß der Definition in einem der Ansprüche 1 bis 8, wobei X und Y unabhägig voneinander für eine -(CH₂)ₘ- Gruppe stehen, wobei m den Wert 1, 2, 3, 4 hat, **dadurch gekennzeichnet, dass** man einen Aminoalkohol mit der Formel II
OH-Z-NH₂ (II),
bei welcher Z die bereits genannte Bedeutung hat, reagieren lässt mit einem Aldehyd der Formel III
GP₁-F₁-V-X'-CHO (III),
bei welcher F₁ und V die bereits genannte Bedeutung haben, GP₁ für eine Schutzgruppe einer Funktion F₁ steht und X' für eine -(CH₂)ₘ₋₁- Gruppe steht, wobei m den bereits genannten Wert hat, zum Erhalten einer Verbindung mit der Formel IV
GP₁-F₁-V-X'-CH₂-NH-Z-OH (IV),
bei welcher GP₁, F₁, V, X' und Z die bereits genannte Bedeutung haben, welche man mit einem Aldehyd der Formel V
GP₂-F₂-W-Y'-CHO (V),
bei welcher F₂ und W die bereits genannte Bedeutung haben, GP₂ für eine Schutzgruppe einer Funktion F2 steht und Y' für eine -(CH₂)ₘ₋₁- Gruppe steht, wobei m den bereits genannten Wert hat, einer reduktiven Aminierung unterzieht um eine Verbindung mit der Formel VI zu erhalten 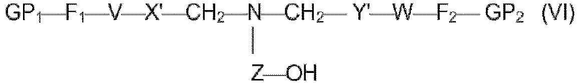 bei welcher GP₁, F₁, V, X', Y', W, F₂, GP₂ und Z die bereits genannte Bedeutung haben, welche man mit einem Alkaliazid mit der Formel VII
EN₃ (VII),
reagieren lässt, bei welcher E für ein Alkalimetall steht, um eine Verbindung mit der Formel VIII zu erhalten 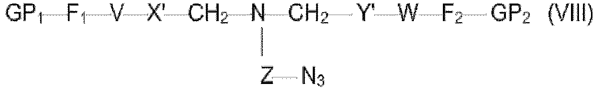 bei welcher GP₁, F₁, V, X', Y', W, F₂, GP₂ und Z die bereits genannte Bedeutung haben, woraus die Schutzgruppen entfernt werden, um die erwartete Verbindung mit der Formel IA zu erhalten 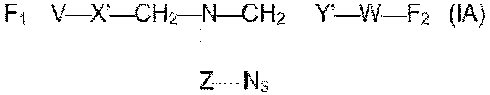 welche, falls gewünscht, isoliert wird.

10. Herstellungsverfahren für ein Azid mit der Formel I gemäß der Definition nach einem der Ansprüche 1 bis 8, wobei V und W für ein Triazol stehen, **dadurch gekennzeichnet, dass** man ein halogeniertes Alkin mit der Formel Xa, 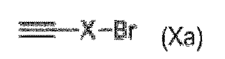 bei welcher X die bereits genannte Bedeutung hat und Br für ein Halogenatom, vorzugsweise ein Brom-Atom steht, mit einem Aminoalkohol mit der Formel XI
OH-Z-NH₂ (XI)
reagieren lässt, bei welcher Z die bereits genannte Bedeutung hat, um ein Dialkin mit der Formel XII zu erhalten 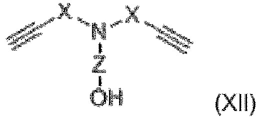 bei welcher X und Y die bereits genannte Bedeutung haben und welches man mit einem Azid mit der Formel XIII
GP₁-F₁-N3 (XIII),
reagieren lässt, bei welcher GP₁ und F₁ die bereits genannte Bedeutung haben, in Anwesenheit eines Katalysators, vorzugsweise auf Kupferbasis, um durch Cycloaddition ein Alkin mit der Formel XIV zu erhalten 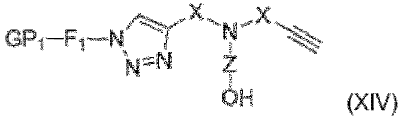 bei welcher GP₁, F₁, X und Z die bereits genannte Bedeutung haben und welches man mit einem Azid mit der Formel XV
GP₂-F₂-N3 (XV)
reagieren lässt, bei welcher GP₂ und F₂ die bereits genannte Bedeutung haben, in Anwesenheit eines Katalysators, vorzugsweise auf Kupferbasis, um durch Cycloaddition eine Verbindung mit der Formel XVI zu erhalten 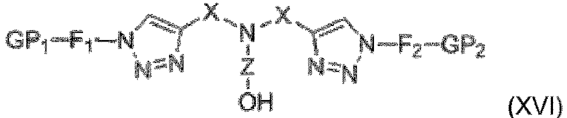 bei welcher GP₁, GP₂, F₁, F₂, X und Z die bereits genannte Bedeutung haben und welches man mit einem Alkaliazid mit der Formel VII
EN₃ (VII)
reagieren lässt, bei welcher E die bereits genannte Bedeutung hat, um eine Verbindung mit der Formel XVII zu erhalten 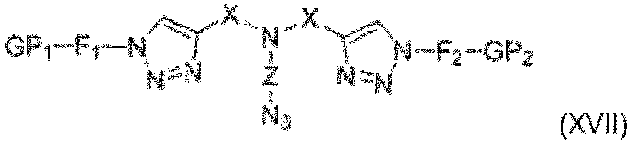 bei welcher GP₁, GP₂, F₁, F₂, X und Z die bereits genannte Bedeutung haben, von welcher die Schutzgruppen entfernt werden, um die Verbindung mit der erwarteten Formel IB zu erhalten 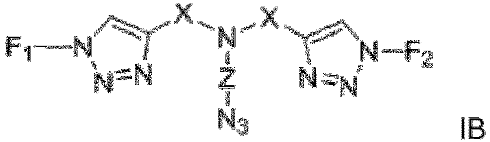 bei welcher F₁, F₂, X und Z die bereits genannte Bedeutung haben und welche, falls gewünscht, isoliert wird.

11. Verwendung eines Azids mit der Formel I gemäß der Definition nach einem der Ansprüche 1 bis 8 für die Zubereitung von Biokonjugaten oder Nanokonjugaten.

12. Verwendung eines Azids mit der Formel I gemäß der Definition nach einem der Ansprüche 1 bis 8 für die Isolierung oder Identifizierung eines Protein-Targets.

13. Verwendung eines Azids mit der Formel I gemäß der Definition nach einem der Ansprüche 1 bis 8 in der Bildgebung.

14. Verwendung nach Anspruch 12 für die Isolierung oder Identifizierung eines Protein-Targets in einer Zelle.

## Claims

1. An azide of formula I 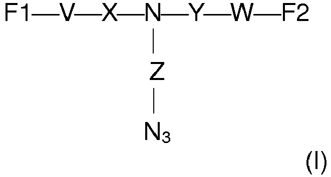 wherein:
V and W represent, independently of one another, a nitrogenous or sulfur-containing heterocycle comprising a copper-complexing heteroatom, said heterocycle being saturated or unsaturated,
X and Y bonded to the central nitrogen represent, independently of one another, a spacer group; -(CH₂)₂-O-(CH₂)-, - (CH₂) -O-(CH₂)₂-, -(CH₂) -O-(CH₂)-, -(CH₂)₂ -S-(CH₂) -, - (CH₂) -S-(CH₂)₂-, -(CH₂)-S-(CH₂)-, -(CH₂)₂-NH-(CH₂)-, -(CH₂)-NH-(CH₂)₂--(CH₂) -NH-(CH₂) -, ou -(CH₂)ₘ -, wherein m can take the values 1, 2, 3 or 4;
Z represents a spacer group -(CH₂)₂ -O-(CH₂) -, -(CH₂) -O-(CH₂)₂-, -(CH₂)₂ -S-(CH₂)-, -(CH₂) -S-(CH₂)₂-, -(CH₂)₂-NH-(CH₂)--(CH₂) -NH-(CH₂)₂ - ou, -(CH₂)ₙ-, wherein n can take the values 1, 2, 3 or 4;
F1 and F2 represent, independently of one another, a hydrogen atom, or a tertiary alkyl group having at most 7 carbon atoms, or a functional group which allows the coupling of the azide of formula I to a chemical molecule, to a biomolecule, to a nanoparticle or to a polymer, said functional group being an isocyanate, isothiocyanate, carboxyl, amino, maleimide or thiol group, indirectly grafted onto the heterocycles, it being understood that at least one of F1 and F2 represents a functional group.

2. The azide of formula I as claimed in claim 1, **characterized in that** X represents a -(CH₂)ₘ - group, wherein m can take the values 1, 2, 3 or 4.

3. The azide of formula I as claimed in claim 1 or 2, **characterized in that** Y represents a -(CH₂)ₘ - group, wherein m can take the values 1, 2, 3 or 4.

4. The azide of formula I as claimed in one of claims 1 to 3, **characterized in that** Z represents an ethylene, propylene or butylene group.

5. The azide of formula I as claimed in one of claims 1 to 4, **characterized in that** V and W represent, independently of one another, a nitrogenous heterocycle.

6. The azide of formula I as claimed in one of claims 1 to 4, **characterized in that** at least one of F1 and F2 represents, independently of the other, a tertiary alkyl group having at most 7 carbon atoms.

7. The azide of formula I as claimed in claim 6, **characterized in that** F1 and F2 represent, each independently of the other a tert-butyl group.

8. The azide of formula I as claimed in claim 1, chosen from
- 5,5'-(4,4'-(((3-azidopropyl)azanediyl)bis(methylene))bis(1H-1,2,3-triazole-4,1-diyl))dipentanoic acid,
- 5-(4-(((3-azidopropyl)((1-(tert-butyl)-1H-1,2,3-triazol-4-yl)methyl)amino)methyl)-1H-1,2,3-triazol-1-yl) pentanoic acid and
- 4,4'-(4,4'-(((3-azidopropyl)azanediyl)bis(methylene))bis(1H-1,2,3-triazol-4,1-diyl))bis(N-(3-aminopropyl)butanamide).

9. A process for preparing an azide of formula I as defined in one of claims 1 to 8, wherein X and Y represent, independently of one another, a -(CH₂)ₘ - group, wherein m has the value 1, 2, 3 or 4, **characterized in that** an amino alcohol of formula II
OH-Z-NH₂ (II)
wherein Z has the meaning already indicated, is reacted with an aldehyde of formula III
GP₁-F₁-V-X'-CHO (III)
wherein F₁ and V have the meaning already indicated, GP₁ represents a group which protects an F1 function and X' represents a -(CH₂)ₘ₋₁- group wherein m has the value already indicated, so as to obtain a compound of formula IV
GP₁-F₁-V-X'-CH₂NH-Z-H (IV)
wherein GP₁, F₁, V, X' and Z have the meaning already indicated,
which is subjected, with an aldehyde of formula V
GP₂-F₂-W-Y'-CHO (V)
wherein F₂ and W have the meaning already indicated, GP₂ represents a group which protects an F2 function and Y' represents a -(CH₂)ₘ₋₁- group wherein m has the value already indicated, to a reductive amination so as to obtain a compound of formula VI 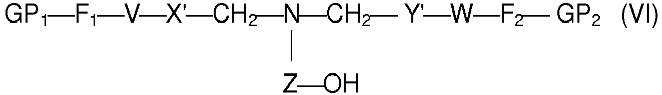 wherein GP₁, F₁, V, X', Y', W, F₂, GP₂ and Z have the meaning already indicated, which is reacted with an alkali azide of formula VII
EN₃ (VII)
wherein E represents an alkali metal, so as to obtain a compound of formula VIII 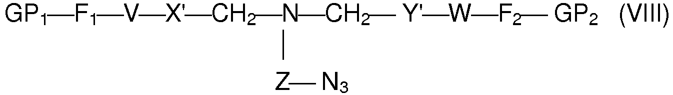 wherein GP₁, F₁, V, X', Y', W, F₂, GP₂ and Z have the meaning already indicated, from which the protective groups are removed so as to obtain the expected compound of formula IA 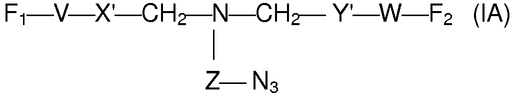 which is isolated if desired.

10. A process for preparing an azide of formula I as defined in one of claims 1 to 8, wherein V and W represent a triazole, **characterized in that** a halogenated alkyne of formula Xa 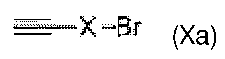 wherein X has the meaning already indicated, and Br represents a halogen atom, preferably a bromine atom, is reacted with an amino alcohol of formula XI
OH-Z-NH₂ (XI)
wherein Z has the meaning already indicated, so as to obtain a dialkyne of formula XII 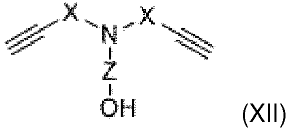 wherein X and Z have the meaning already indicated, which is reacted with an azide of formula XIII
GP₁-F₁-N₃ (XIII)
wherein GP₁ and F₁ have the meaning already indicated, in the presence of a catalyst, preferably a copper-based catalyst, so as to obtain by cycloaddition an alkyne of formula XIV, 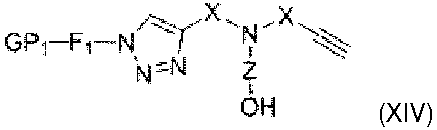 wherein GP₁, F₁, X and Z have the meaning already indicated, which is reacted with an azide of formula XV
GP₂-F₂-N₃ (XV)
wherein GP₂ and F₂ have the meaning already indicated, in the presence of a catalyst, preferably a copper-based catalyst, so as to obtain by cycloaddition a compound of formula XVI, 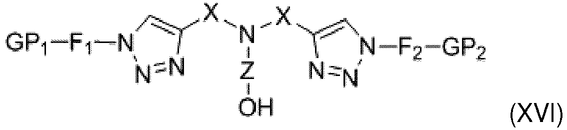 wherein GP₁, GP₂, F₁, F₂, X and Z have the meaning already indicated, which is reacted with an alkali azide of formula VII
EN₃ (VII)
wherein E has the meaning already indicated, so as to obtain a compound of formula XVII 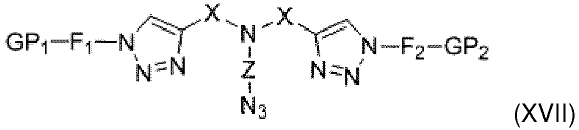 wherein GP₁, GP₂, F₁, F₂, X and Z have the meaning already indicated, from which the protective groups are removed so as to obtain the expected compound of formula IB 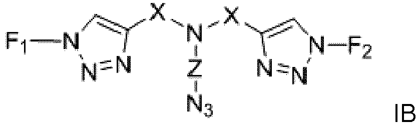 wherein F₁, F₂, X and Z have the meaning already indicated, which is isolated if desired.

11. Use of an azide of formula I as defined in one of claims 1 to 8, for the preparation of bioconjugates or of nanoconjugates.

12. Use of an azide of formula I as defined in one of claims 1 to 8, for the isolation or identification of a protein target.

13. Use of an azide of formula I as defined in one of claims 1 to 8, in imaging.

14. Use as claimed in claim 12 for the isolation or identification of a protein target within a cell.
